# EUROPEAN PATENT SPECIFICATION

(11) **EP 0 653 065 B1**
(45) Date of publication and mention of the grant of the patent: **30.10.2002**
(21) Application number: 93917967.7
(22) Date of filing: 02.08.1993
(51) Int. Cl.: G01N 33/537, G01N 33/543, G01N 33/58

(54) **SEPARATION METHOD**
TRENNVERFAHREN
PROCEDE DE SEPARATION

(30) Priority: 03.08.1992 GB 9216450; 06.08.1992 GB 9216683; 18.09.1992 GB 9219743; 01.10.1992 GB 9220722; 27.11.1992 GB 9224898; 27.11.1992 GB 9224897
(43) Date of publication of application: 17.05.1995
(73) Proprietor: Marconi Optical Components Limited, London WIX 8AQ (GB)
(72) Inventor: ABUKNESHA, Ramadan, Arbi 16 Burgess Park Mansions, London NW6 1DP (GB)
(74) Representative: Cockayne, Gillian
(86) International application number: GB9301627
(87) International publication number: WO94003807

(56) References cited:
- EP-A- 0 177 191
- EP-A- 0 188 093
- EP-A- 0 538 053
- WO-A-92/16838

## Description

The present invention relates to a separation method which finds application in immunological detection (e.g. in immunoassay and in immunosensors), to a method suitable for use in detection, to a sensor, to apparatus for use in detection, and to a test-kit.

According to one aspect of the present invention there is provided a separation method, suitable for use in an immunological method for the detection of a plurality of entities, which separation method includes using a plurality of different auxiliary ligand-binder pairs, the auxiliary ligand of each of the plurality of auxiliary ligand-binder pairs being provided on a support material, said auxiliary ligands not being primary species, said separation method being such that immunological specific binding interaction between a given auxiliary ligand and a binder therefor facilitates association of a primary species with a support material.

A binder of a given auxiliary ligand-binder pair is a species capable of undergoing a specific binding reaction with the auxiliary ligand of the said given auxiliary ligand-binder pair. Where, for example, the binder is an auxiliary species, as is further disclosed hereinafter, the binder may be an auxiliary binder as is also further disclosed hereinafter. Alternatively, for example, the binder may be a species which has a part which is capable of undergoing a specific binding reaction with the auxiliary ligand and a part which is capable of undergoing a specific binding reaction with a primary species, as is further disclosed hereinafter.

In accordance with one embodiment of the present invention there is provided a separation method, suitable for use in an immunological method for the detection of a plurality of entities, which separation method includes using a plurality of different auxiliary ligand-auxiliary binder pairs, the auxiliary ligands and the auxiliary binders of the pairs not being primary species, and the auxiliary ligand of each of the plurality of auxiliary ligand-auxiliary binder pairs being provided on a support material.

According to a further aspect of the present invention there is provided a method, suitable for use in immunological detection of a plurality of entities, which method includes using a plurality of different auxiliary ligand-binder pairs, the auxiliary ligand of each of the plurality of auxiliary ligand-binder pairs being provided on a support material, said auxiliary ligands not being primary species, said method being such that immunological specific binding interaction between a given auxiliary ligand and a binder therefor facilitates association of a primary species with a support material.

In accordance with another embodiment of the present invention there is provided a method, suitable for use in immunological detection of a plurality of entities, which method includes using a plurality or different auxiliary ligand-auxiliary binder pairs, the auxiliary ligands and the auxiliary binders of the pairs not being primary species, and the auxiliary ligand of each of the plurality of auxiliary ligand-auxiliary binder pairs being provided on a support material.

By way of example, an auxiliary ligand of an auxiliary ligand-binder pair (e.g. an auxiliary ligand of an auxiliary ligand-auxiliary binder pair), may be provided on a support material as is further disclosed hereinafter.

According to a further aspect of the present invention there is provided a sensor, suitable for use in immunological detection of a plurality of entities, which sensor includes a support material there being provided on the support material when the sensor is in use a plurality of different auxiliary ligands, said auxiliary ligands not being primary species, the arrangement being such that, when the sensor is in use, immunological specific binding interaction between a given auxiliary ligand and a binder therefor facilitates association of a primary species with the support material.

An auxiliary ligand-binder pair (e.g. auxiliary ligand-auxiliary binder pair) is such that the auxiliary ligand and binder of the pair interact so as to bind together by an immunological specific binding reaction to effect a separation in accordance with the present invention.

It is to be understood that a separation method in accordance with the present invention may find application in, for example, separation of fractions in an immunoassay method.

By use of a plurality of different auxiliary ligand-binder pairs (i.e. a plurality of different kinds of pairs of auxiliary ligand and binder (e.g. a plurality of different kinds of auxiliary ligand-auxiliary binder pairs)), a plurality of different specific separations may be effected. Accordingly, a separation method in accordance with the present invention may involve a plurality of different specific separations. Thus, for example, by use of a plurality of auxiliary ligand-binder pairs (e.g. a plurality of auxiliary ligand-auxiliary binder pairs) in accordance with the present invention a plurality of different entities may be detected. Accordingly, the present invention may be utilised, for example, to effect "multi-entity" detection (e.g. "multi-analyte species" detection), as is further disclosed hereinafter.

Various specificities may be relevant to the capability of carrying out a plurality of specific separations in accordance with the present invention, and in carrying out multi-entity detection in accordance with the present invention; such specificities are further discussed hereinafter.

where, for example, an auxiliary ligand-binder pair comprises an auxiliary ligand-auxiliary binder pair, such a pair in accordance with the present invention may be regarded as "auxiliary" in that they do not take part in a primary immune binding reaction. It will be appreciated that an "auxiliary species", comprising an auxiliary ligand or an auxiliary binder, does not, itself, take part in a primary immune binding reaction.

By way of example, a primary immune binding reaction in accordance with the present invention is one in which an entity to be detected undergoes a specific binding reaction, or an authentic entity to be detected (as hereinafter defined) undergoes, a specific binding reaction, or an entity to be detected and an authentic entity to be detected undergo specific binding reactions. (It will be appreciated that the entity to be detected and the authentic entity to be detected undergo specific binding reactions with other species and not with each other.)

It is to be understood that an "authentic entity" is an entity which is capable of reacting in a substantially similar manner as an entity to be detected under substantially similar conditions. Further, it is to be understood that an authentic entity may, for example, have a chemically defined structure and/or a biologically defined activity.

It will be appreciated that an authentic entity to be detected may be used, for example, as a "standard" or a "calibrator". Where, for example, the entity to be detected is itself an analyte species (as hereinafter disclosed) the authentic entity may be an authentic analyte species (or a suitable derivative thereof).

Where, for example, a binder of an auxiliary ligand-binder pair is a species which has a part which is capable of undergoing a specific binding reaction with the auxiliary ligand and a part which provides a primary species (said primary species being a binder for a primary species) which is capable of undergoing a specific binding reaction with a primary species it will be understood that the part capable of undergoing specific binding with the auxiliary ligand may be regarded as an "auxiliary function". An example of a species which has a part capable of undergoing a specific binding reaction with an auxiliary ligand and a part which is a primary species capable of undergoing a specific binding reaction with a primary species is a bifunctional antibody.

A binder of an auxiliary ligand-binder pair (e.g. an auxiliary binder of an auxiliary ligand-auxiliary binder pair) may be associated with a primary species as will be further disclosed hereinafter; a primary species in accordance with the present invention is a species capable of taking part in a primary immune binding reaction.

A binder of an auxiliary ligand-binder pair (e.g. an auxiliary binder of an auxiliary ligand-auxiliary binder pair) may be arranged to be associated with a primary species in any suitable manner and at any suitable time. Thus, a binder of an auxiliary ligand-binder pair (e.g. an auxiliary binder of an auxiliary ligand-auxiliary binder pair) may, for example, be arranged to be associated with a primary species either before, or after, binding between the binder (e.g. auxiliary binder) and an auxiliary ligand therefor has taken place.

An auxiliary ligand of an auxiliary ligand-binder pair (e.g. of an auxiliary ligand-auxiliary binder pair) may be a suitable ligand examples of which are antigenic ligands (such as haptens).

Examples of antigenic ligands are 2,4 dinitrophenol, fluorescein, digitoxin, coumarin, cibacron blue (Registered Trade Mark), 2-(4-aminophenyl)-6-methyl benzothiazole-hemiglutarate, camphorcarboxylic acid, 4-amino-benzo-15-crown-5, carboxyfluorescein, 3-methyl-1-adamantane acetic acid, 2-phenyl-4-quinoline carboxylic acid, xanthine-9-carboxyamide-glycine-glycine, 4-hydroxy-7-trifluoromethyl-3-quinaldine carboxylic acid, cis-bicyclo [3,3,0] octane-2-carboxylic acid, endo-bicyclo [2,2,2] oct-5-ene-2,3-dicarboxylic anhydride, N-[4-(4-aminobenzyl) phenyl]-5-norbornene-2,3-dicarboximide, and [IR-(2-endo, 3-exo)]-3-hydroxy-4,7,7-trimethyl bicyclo [2,2,1] heptane-2-acetic acid.

Further examples of auxiliary ligands which may be attached to a support material (e.g. by covalent linkage or non-covalent attachment (adsorption)) are soluble materials for example soluble molecules (e.g. polymers) such as polypeptides, proteins, polysaccharides and conducting polymers; haptenic functions may be covalently coupled to such soluble materials (e.g. polymers). By way of example, an auxiliary ligand may be provided as a coating of a polymer on a support material.

A binder of an auxiliary ligand-binder pair (e.g. an auxiliary binder of an auxiliary ligand-auxiliary binder pair) may be any suitable species capable of binding with an auxiliary ligand.

By way of example, the binder may be a binding protein comprising an antibody.

Thus, for example, a binder for an auxiliary ligand (which is an antigenic ligand) may be an auxiliary binder which is an antibody to the ligand. An antibody to the auxiliary ligand may be considered to be an anti-auxiliary ligand antibody.

Accordingly, where, for example, the auxiliary ligand is 2,4 dinitrophenol, fluorescein, digitoxin, coumarin, cibacron blue, 2-(4-aminophenyl)-6-methyl benzothiazole-hemiglutarate, camphorcarboxylic acid, 4-amino-benzo-15-crown-5, carboxyfluorescein, 3-methyl-1-adamantane acetic acid, 2-phenyl-4-quinoline carboxylic acid, xanthine-9-carboxyamide-glycine-glycine, 4-hydroxy-7-trifluoromethyl-3-quinaldine carboxylic acid, cis-bicyclo [3,3,0] octane-2-carboxylic acid, endo-bicyclo [2,2,2] oct-5-ene-2,3-dicarboxylic anhydride, N-[4-(4-aminobenzyl) phenyl]-5-norbornene-2,3-dicarboximide, or [IR-(2-endo, 3-exo)]-3-hydroxy-4,7,7-trimethyl bicyclo [2,2,1] heptane-2-acetic acid, the binder may be an auxiliary binder which may be, respectively, anti-2,4 dinitrophenol antibody, anti-fluorescein antibody, anti-digitoxin antibody, anti-coumarin antibody, anti-cibacron blue antibody, anti-2-(4-aminophenyl)-6-methyl benzothiazole-hemiglutarate antibody, anti-camphorcarboxylic acid antibody, anti-4-amino-benzo-15-crown-5 antibody, anti-carboxyfluorescein antibody, anti-3-methyl-1-adamantane acetic acid antibody, anti-2-phenyl-4-quinoline carboxylic acid antibody, anti-xanthine-9-carboxyamide-glycine-glycine antibody, anti-4-hydroxy-7-trifluoromethyl-3-quinaldine carboxylic acid antibody, anti-cis-bicyclo [3,3,0] octane-2-carboxylic acid antibody, anti-endo-bicyclo [2,2,2] oct-5-ene-2,3-dicarboxylic anhydride antibody, anti-N-[4-(4-aminobenzyl) phenyl]-5-norbornene-2,3-dicarboximide antibody, or anti-[IR-(2-endo, 3-exo)]-3-hydroxy-4,7,7-trimethyl bicyclo [2,2,1] heptane-2-acetic acid antibody.

It will be appreciated that antibodies such as those immediately hereinbefore disclosed may be prepared by any suitable method, for example those known for the raising of polyclonal or monoclonal antibodies; thus, antibodies may be raised, for example, by immunising animals with conjugates made of suitable derivatives of a ligand and an immunogenic carrier protein such as bovine serum albumin or key-hole limpet haemocyanin; the product obtained by immunising animals may be purified as desired (e.g. by the use of affinity chromatography) to obtain the required antibodies.

In view of the foregoing disclosure it may be appreciated that a separation method in accordance with the present invention may, for example, utilise separation systems comprising auxiliary ligand-auxiliary binder pairs such as 2,4 dinitrophenol/anti-2,4 dinitrophenol antibody, fluorescein/anti-fluorescein antibody, digitoxin/anti-digitoxin antibody, coumarin/anti-coumarin antibody, cibacron blue/anti-cibacron blue antibody, 2-(4-aminophenyl)-6-methyl benzothiazole-hemiglutarate/anti-2-(4-aminophenyl)-6-methyl benzothiazole-hemiglutarate antibody, camphorcarboxylic acid/anti-camphorcarboxylic acid antibody, 4-amino-benzo-15-crown-5/anti-4-amino-benzo-15-crown-5 antibody, carboxyfluorescein/anti-carboxyfluorescein antibody, 3-methyl-1-adamantane acetic acid/anti-3-methyl-1-adamantane acetic acid antibody, 2-phenyl-4-quinoline carboxylic acid/anti-2-phenyl-4-quinoline carboxylic acid antibody, xanthine-9-carboxyamide-glycine-glycine/anti-xanthine-9-carboxyamide-glycine-glycine antibody, 4-hydroxy-7-trifluoromethyl-3-quinaldine carboxylic acid/anti-4-hydroxy-7-trifluoromethyl-3-quinaldine carboxylic acid antibody, cis-bicyclo [3,3,0] octane-2-carboxylic acid/anti-cis-bicyclo [3,3,0] octane-2-carboxylic acid antibody, endo-bicyclo [2,2,2] oct-5-ene-2,3-dicarboxylic anhydride/anti-endo-bicyclo [2,2,2] oct-5-ene-2,3-dicarboxylic anhydride antibody, N-[4-(4-aminobenzyl) phenyl]-5-norbornene-2,3-dicarboximide/anti-N-[4-(4-aminobenzyl) phenyl]-5-norbornene-2,3-dicarboximide antibody, or [IR-(2-endo, 3-exo)]-3-hydroxy-4,7,7-trimethyl bicyclo [2,2,1] heptane-2-acetic acid/anti-[IR-(2-endo, 3-exo)]-3-hydroxy-4,7,7-trimethyl bicyclo [2,2,1] heptane-2-acetic acid antibody.

In one embodiment of the present invention, auxiliary ligands of different auxiliary ligand-binder pairs may be provided on a support material such that, upon binding occurring between the auxiliary ligands and their respective corresponding binders of the particular pairs, the respective corresponding binders become associated with the support material; thus, it will be appreciated that, in accordance with the immediately foregoing embodiment of the present invention, any primary species associated with the binders will also become associated with the support material upon binding occurring between the auxiliary ligands and the binders thereby to effect a separation in accordance with the present invention.

In accordance with another embodiment of the present invention, auxiliary ligands of particular auxiliary ligand-auxiliary binder pairs may be provided on a support material such that, upon binding occurring between the auxiliary ligands and their respective corresponding auxiliary binders of the particular pairs, the respective corresponding auxiliary binders become associated with the support material; it will be appreciated that, in accordance with the immediately foregoing embodiment of the present invention, any primary species associated with the auxiliary binders will also become associated with the support material upon binding occurring between the auxiliary ligands and the auxiliary binders thereby to effect a separation in accordance with the present invention.

By way of example, in accordance with an embodiment of the present invention, a plurality of different regions or zones of a support material may be arranged such that a selected specific separation may be effected at a given region or zone.

Thus, by way of example, different regions or zones of a support material may be arranged to provide different auxiliary ligands such that by use of appropriate binders (e.g. auxiliary binders) different specific separations may be effected on different regions or zones of the support material.

By way of example, it will be appreciated that in accordance with the present invention, any suitable number of different auxiliary ligands may be provided on a support material.

Thus, for example, a plurality of different auxiliary ligands may be provided on a support material.

From the foregoing disclosure it will be appreciated that the present invention may be utilised, for example, to effect a first selected separation at a first region of a support material, a second selected separation at a second region of a support material, and so forth using as many regions as required to effect a desired number of separations. It is to be understood that the number of separations it is desired to effect may be determined by the number of entities which it is desired to "probe" for in a given application.

By way of example, a support material having a plurality of regions or zones for effecting a plurality of specific separations may be contacted with a reaction mixture in what may be termed a "simultaneous" mode of operation (i.e a reaction mixture may be exposed to a support material in one operation).

Thus, for example, in a "simultaneous" mode of operation in accordance with the present invention, a plurality of auxiliary ligands may be provided on a given support material.

Alternatively, by way of example, a reaction mixture may be contacted sequentially with a set of support materials each of which support materials is capable of effecting a specific separation which is different from that which may be effected by another member of the set. Thus, upon sequential contacting of a reaction mixture with members of the set, different entities (if present in a sample) will appear on different support materials.

Thus, members of the set may act as "probes" for particular entities.

Accordingly, for example, a "sequential" mode of operation may be utilised in accordance with the present invention.

By way of example, where a set of support materials is used to effect a "sequential" mode of operation, any suitable selection of auxiliary ligands may be utilised. Thus, for example, each member of the set may be provided with an auxiliary ligand (e.g. each member may provide a different auxiliary ligand).

By way of further example, rather than using a set of separate support materials, a single support material may provide a plurality of regions to which a reaction mixture may be introduced sequentially (e.g. by use of a support material in the form of a wick, or similar arrangement, having regions that a reaction mixture meets sequentially as it travels along the support material). Each of the regions may provide an auxiliary ligand (e.g. each region may provide a different auxiliary ligand).

Thus, according to a further aspect of the present invention there is provided a sequential separation method, suitable for use in an immunological method for the detection of a plurality of entities, which sequential separation method includes using a plurality of different auxiliary ligand-binder pairs, said separation method being such that specific binding interaction between a given auxiliary ligand and a binder therefor facilitates association of a primary species with a support material.

By way of example, the auxiliary ligand-binder pairs in a sequential separation method in accordance with the invention may include an auxiliary ligand-auxiliary binder pair, or each of the auxiliary ligand-binder pairs may be an auxiliary ligand-auxiliary binder pair.

According to a further aspect of the present invention there is provided a method, suitable for use in immunological detection of a plurality of entities, which method includes the use of a sequential separation method in accordance with the present invention.

Entities to be detected may, for example, be detected by any suitable means, such as those known in the art, whilst still associated with a support material; alternatively, by way of example, entities to be detected may be eluted from a support (e.g. using suitable buffer solutions) and then subject to detection.

Examples of support materials which may find application in accordance with the present invention are solid phase materials such as a reaction vessel wall, insoluble polysaccharides, microparticles (e.g. particulate microcellulose), polystyrene (e.g. in the form of wells, beads, microtitre plates, discs, sticks or tubes), cross-linked dextran (e.g. Sephadex (Registered Trade Mark)), insoluble polymer structures, glass surfaces, derivatised silica surfaces (e.g. having silyl groups with chemical functions attached), soluble polymers attached to a suitable surface (e.g. a glass surface), microparticulate materials with entrapped ferrous oxide (magnetisable particles), nylon and polyamides.

By way of further example, a support material may be in the form of a carrier (e.g. a tape) which may be moved from a sample application means, for applying a sample to the carrier, to a detection means thereby to allow successive (e.g. continuous) detection for a plurality of different entities; thus, for example, successive (e.g. continuous) multi-entity detection (e.g. multi-analyte species detection) may be effected.

It will be appreciated that some types of support materials may be inappropriate for use with some entities to be detected.

Thus, for example, where the entity to be detected is a metal ion, or contains a metal, the use of some types of support materials may be inappropriate (e.g. support materials containing entrapped iron oxide may give rise to unacceptable interference).

Also, it is to be understood that some types of support materials may not be appropriate for some types of detection in accordance with the present invention. Thus, for example, particulate support materials may not be appropriate where a plurality of zones, each providing a different auxiliary ligand, or a different auxiliary binder, is required on a support material.

An auxiliary ligand may be, for example, provided on a support material in any suitable manner, and it is to be understood that in this specification "providing on a support material" and "provided on a support material" embrace, for example, situations where the support material carries an auxiliary ligand, and situations where the support material itself, or a part of the support material itself, provides an auxiliary ligand.

Thus, for example, an auxiliary ligand may be provided by chemical groups or units of the support material. Alternatively, an auxiliary ligand may be, for example, attached to the support material (e.g. by covalent linkage or adsorption). Where the support material is, for example, a polymer, units of the polymer may act as an auxiliary ligand. Also, by way of example, surface groups present on a support material, such as polystyrene or modified silica, may act as an auxiliary ligand.

By way of example, the support material may, if desired, provide oligomers or polymers of an auxiliary ligand.

Where an auxiliary ligand is attached to a support material the auxiliary ligand may be directly attached to the support material or indirectly attached to the support material via other species (e.g. a carrier protein).

In one embodiment the present invention provides a method which also includes the step of attaching, either directly, or indirectly, an auxiliary ligand to a support material.

By way of further example, the surface of a support material may be activated thereby to permit attachment of auxiliary ligand; for example, the surface of a suitable support material may be activated by chemical treatment to provide free amino groups to which an auxiliary ligand may be linked.

Further, by way of example, an oligomer or oligomers of an auxiliary ligand, or a polymer or polymers of an auxiliary ligand, may be attached directly or indirectly to a support material.

Thus for example, oligomers of an auxiliary ligand or polymers of an auxiliary ligand may be attached to free amino groups on a support material.

Also, by way of example, an auxiliary ligand may be linked (e.g. covalently or otherwise) to a further species (e.g. a carrier protein or a polymer) and the further species may be associated with the support material such that the auxiliary ligand may become indirectly provided on the support material.

By way of further example, it is possible to link (e.g. covalently or otherwise) oligomers or polymers of auxiliary ligand to a further species (e.g. a carrier protein or a polymer) and the further species may be associated with the support material such that oligomers of auxiliary ligand or polymers of auxiliary ligand may become indirectly provided on the support material.

Where an auxiliary ligand is attached to a support material, attachment of the auxiliary ligand may be effected at any desired time. Thus, for example, an auxiliary liqand may be allowed to bind with an appropriate binder (e.g. an auxiliary binder) before, or after, the auxiliary ligand is attached to the support material; however, in general, it may be more convenient to attach an auxiliary ligand to the support material before bringing together the auxiliary ligand and a binder (e.g. auxiliary binder).

It is to be understood that the use of oligomers or polymers of auxiliary ligands or of auxiliary binders may be advantageous in certain circumstances. Thus, for example, by use of an oligomer or a polymer of an auxiliary ligand or an auxiliary binder more auxiliary ligand or auxiliary binder may be provided than it is possible to provide when using only single "units" of auxiliary ligand or auxiliary binder. Accordingly, for example, the provision of more auxiliary ligand or auxiliary binder offers the possibility of faster reactions since more auxiliary ligand or auxiliary binder is available to undergo reaction, respectively, with available auxiliary binder or auxiliary ligand.

A given binder (e.g. an auxiliary binder) may be arranged to bind with a given auxiliary ligand or a given auxiliary ligand may be arranged to bind with a given binder (e.g. an auxiliary binder), in any suitable manner and at any suitable time. Thus, for example, a binder (e.g. an auxiliary binder) may be arranged to be linked (either directly or indirectly) with a primary species either before, or after, binding with an auxiliary ligand.

It is to be understood that an auxiliary binder may be associated with a primary species by any suitable linkage; such a linkage may include a non-specific binding link or links (e.g. a covalent link or links or adsorption) or a specific binding link or links, or any combination of such links.

By way of example, a binder (e.g. an auxiliary binder) may be arranged to be linked, either directly or indirectly, with a primary species by means of any suitable linkage. An example of such a linkage is one which involves a link, of a non-specific binding type, to the auxiliary binder.

An example of a link of a non-specific binding type is a covalent link. Thus, for example, the linkage may involve a covalent link to an auxiliary binder.

Another example of a link of a non-specific binding type is a link which involves adsorption. Thus, for example, an auxiliary binder and a primary species may be linked by both being adsorbed on a suitable material (e.g. a carrier material such as latex particles).

As hereinbefore disclosed an auxiliary binder may be linked with a primary species directly or indirectly (e.g. via other species).

Thus, for example, where an auxiliary binder is arranged to be linked directly to a primary species the linkage may be a link of a non-specific binding type (e.g. a covalent link) between the auxiliary binder, and the primary species.

By way of further example, where an auxiliary binder is arranged to be linked indirectly to a primary species the linkage may include one or more other species, and one or more links, as required, one of the links being a link, of a non-specific binding type, to the auxiliary binder.

It is to be understood that where, for example, one or more links are involved in the linkage, and one of the links is a link of a non-specific binding type to the auxiliary binder, any further link or links in the linkage may be of any suitable type (e.g. non-specific binding type or specific binding type); thus, for example, the linkage may include a further link or links of a non-specific binding type, or may include a further link or links of a specific binding type (e.g. ligand-binder type), or a mixture of types of links (e.g. non-specific binding type and specific binding type).

Where an auxiliary binder is linked to a primary species via one or more other species, said other species or one of said other species may be, for example, a second antibody or a ligand or a binder and, for example, the auxiliary binder may be covalently linked to said other species or said one of said other species.

By way of example, a primary species may be a primary antibody or a ligand (e.g. an antigen). It is to be understood that, for example, a primary species may be an antibody to an entity to be detected or an antibody to an authentic entity to be detected; it will be appreciated that, for a given immunoassay, the antibody to the entity to be detected and the antibody to the authentic entity to be detected will be the same antibody. It is also to be understood that a primary species may be, for example an entity to be detected or an authentic entity to be detected.

The term "antibody" as used in this Specification embraces whole antibody or antibody fragments such as Fab and (Fab)₂ and, accordingly, the term "antibodies" used herein embraces whole antibodies and antibody fragments.

It will be appreciated that, for example, a primary species, comprising an antibody to an entity to be detected, may be prepared by any suitable method, for example those known for raising antibodies as hereinbefore disclosed.

The present invention may find application in any suitable form of immunological detection or immunoassay examples of which are heterogeneous immunoassay methods, competitive immunoassay methods, homogeneous immunoassay methods, non-competitive immunoassay methods, sandwich immunoassay methods and direct immunoassay methods.

The present invention may find application in, for example, label-free or detectable species-dependent (e.g. tracer species-dependent) assay methods such as enzyme-immunoassay, fluoro-immunoassay and radio-immunoassay. By way of example, the present invention may find application in antibody-labelled or antigen-labelled assays.

The present invention may find application in any suitable assay configuration. Thus, for example, the present invention may find application in:
(a) competitive binding assays (either ligand-labelled or antibody labelled),
(b) non-competitive sandwich assays,
(c) assays wherein specifically extractable labelled complexes of entities are involved.

By way of example, when applying the present invention with any of the assay types immediately hereinbefore disclosed in (a) to (c) a plurality of auxiliary ligands may be provided on a support material.

Thus, the present invention provides, for example, immunological detection or immunoassay which includes a separation method in accordance with the present invention.

By way of example, any suitable detectable species may be used in accordance with the present invention in the detection of an entity to be detected. It is to be understood that a detectable species may be, for example, a detectable structure.

An auxiliary ligand may, for example, provide a detectable species.

Where an auxiliary ligand provides a detectable species the auxiliary ligand may provide a detectable species at any suitable time and in any suitable manner.

Thus, for example, an auxiliary ligand, or a part thereof (which part may be, for example, a structure comprising a chemical group or combination of chemical groups) may be a detectable species (e.g. a detectable structure); where, for example, an auxiliary ligand, or a part thereof, is a detectable species a ratiometric detection may be carried out.

Alternatively, for example, an auxiliary ligand or a binder (e.g. an auxiliary binder) may be capable of being associated with a detectable species, and the auxiliary ligand or the binder (e.g. auxiliary binder) may be associated with the detectable species at any suitable time and in any suitable manner.

By way of example, an auxiliary ligand or a binder (e.g. an auxiliary binder) may be associated with a detectable species at any convenient time during the performance of a method in accordance with the present invention.

Also, by way of further example, an auxiliary ligand or a binder (e.g. an auxiliary binder) may be associated directly with a detectable species (e.g. by means of a suitable linkage such as a covalent bond); alternatively an auxiliary ligand or a binder (e.g. an auxiliary binder) may be indirectly associated with a detectable species (e.g. via one or more other species).

Enzymes and species capable of giving a fluorescence signal (e.g. fluorophores) are examples of species which may be chosen so as to act both as an auxiliary ligand and a detectable species.

Examples of detectable species are enzymes (e.g. alkaline phosphatase, β-galactosidase and horse-radish peroxidase), species capable of giving a fluorescent signal (e.g. fluorophores (or polymeric fluorophores)), chemiluminescent compounds, bioluminescent compounds, radioisotopes, dyes, ligand species (or polymers of a ligand species), and binder species (or polymers of binder species. Examples of fluorophores are fluoresceins, coumarins and rhodamine.

By way of example, an enzyme may be detected by a corresponding substrate, fluorophores and radioisotopes may be detected directly with suitable detectors, ligand species may be detected by use of binder species therefor, said binder species being associated with tracer species, binder species may be detected by use of ligand species therefor, said ligand species being associated with tracer species.

The tracer species may be, for example, any suitable tracer species such as those known in the art relating to protein binding assays (e.g. immunoassays). (It is to be understood that a tracer species may also be considered to be a signal species or a labelling species.)

Examples of tracer species are enzymes (e.g. alkaline phosphatase, β-galactosidase and horse-radish peroxidase), species capable of giving a fluorescent signal (e.g. fluorophores (e.g. fluoresceins, coumarins or rhodamine)), chemiluminescent compounds, bioluminescent compounds, radioisotopes and dyes.

Detection or measurement of a signal from a detectable species may be carried out in any suitable manner such as those known in the immunochemical field.

Where, for example, a ligand species or a binder species is used (as hereinbefore disclosed) as a detectable species any suitable ligand species or binder species may be utilised.

Examples of ligand species which may be utilised as detectable species are antigenic species (e.g. 2,4 dinitrophenol, fluorescein, digitoxin, coumarin, cibacron blue, 2-(4-aminophenyl)-6-methyl benzothiazole-hemiglutarate, camphorcarboxylic acid, 4-amino-benzo-15-crown-5, carboxyfluorescein, 3-methyl-1-adamantane acetic acid, 2-phenyl-4-quinoline carboxylic acid, xanthine-9-carboxyamide-glycine-glycine, 4-hydroxy-7-trifluoromethyl-3-quinaldine carboxylic acid, cis-bicyclo [3,3,0] octane-2-carboxylic acid, endo-bicyclo [2,2,2] oct-5-ene-2,3-dicarboxylic anhydride, N-[4-(4-aminobenzyl) phenyl]-5-norbornene-2,3-dicarboximide, and [IR-(2-endo, 3-exo)]-3-hydroxy-4,7,7-trimethyl bicyclo [2,2,1] heptane-2-acetic acid), which may be considered to be haptens, and non-antigenic ligand species such as the ligand species of a specific ligand species-binder species pair (e.g. biotin).

Examples of binder species which may be utilised as detectable species are antibodies (e.g. anti-2,4 dinitrophenol antibody, anti-fluorescein antibody, anti-digitoxin antibody, anti-coumarin antibody, anti-cibacron blue antibody, anti-2-(4-aminophenyl)-6-methyl benzothiazole-hemiglutarate antibody, anti-camphorcarboxylic acid antibody, anti-4-amino-benzo-15-crown-5 antibody, anti-carboxyfluorescein antibody, anti-3-methyl-1-adamantane acetic acid antibody, anti-2-phenyl-4-quinoline carboxylic acid antibody, anti-xanthine-9-carboxyamide-glycine-glycine antibody, anti-4-hydroxy-7-trifluoromethyl-3-quinaldine carboxylic acid antibody, anti-cis-bicyclo [3,3,0] octane-2-carboxylic acid antibody, anti-endo-bicyclo [2,2,2] oct-5-ene-2,3-dicarboxylic anhydride antibody, anti-N-[4-(4-aminobenzyl) phenyl]-5-norbornene-2,3-dicarboximide antibody, and anti-[IR-(2-endo, 3-exo)]-3-hydroxy-4,7,7-trimethyl bicyclo [2,2,1] heptane-2-acetic acid antibody) and binder species of a specific ligand species-binder species pair (e.g. avidin). It will be appreciated that antibodies such as those immediately hereinbefore disclosed may be prepared by any suitable method such as those known for the raising of polyclonal or monoclonal antibodies; thus antibodies may be raised, for example, by immunising animals.

By way of further example, a detectable species comprising an enzyme may be detected by means of an enzyme induced change.

The enzyme induced change may be, for example, a colour change, or a fluorescence change, or a luminescence change, or an electro-chemical change.

Where a detectable species is used in accordance with the present invention, the detectable species may be, for example, arranged to be associated, in any suitable manner and at any suitable time, with any chosen species or entity; thus, for example, a detectable species may be associated (as appropriate to an immunoassay being utilised) with an authentic entity (being an authentic entity to be detected) or with a primary antibody, or with a second antibody.

It is to be understood that a separation method in accordance with the present invention may be used, for example, in the separation of antibody bound and unbound fractions of a detectable species in an immunoassay method.

Entities to be detected may be, for example, any suitable entities capable of undergoing a specific binding reaction.

Thus, for example, entities to be detected may themselves be analyte species. For example, entities to be detected may be analyte species comprising ligands (e.g. antigenic ligands such as haptens). By way of further example, entities to be detected may be analyte species comprising antibodies.

Examples of entities which are analyte species which may be detected in accordance with the present invention are:
(a) steroid hormones such as progesterone, 17α-hydroxy progesterone or estradiol (e.g. in a sample of blood, serum, saliva, urine or milk),
(b) hormones such as thyroid hormone (e.g. thyroxine or triiodothyronine),
(c) steroids in extracts (e.g. extracts of solids or liquids),
(d) drugs such as drugs of abuse (e.g. phenobarbital) and therapeutic drugs (e.g. digoxin) (in for example, a sample of blood, serum, saliva or urine),
(e) polypeptide hormones (e.g. hCG) in, for example, a sample of blood or urine,
(f) tumour markers such as marker proteins (e.g. in a sample of blood or serum),
(g) protein antigens,
(h) blood proteins (e.g. human serum albumin, immunoglobulins (e.g. IgG), enzyme markers or receptors),
(i) marker proteins in urine resulting from kidney diseases,
(j) pesticides such as insecticides, or herbicides (e.g. in water or soil),
(k) toxins (such as those extracted from feeds and food stuffs),
(l) micro-organisms (e.g. viruses and bacteria), and
(m) antibodies to micro-organisms.

Further examples of entities which are analyte species which may be detected in accordance with the present invention are complexes of metals.

Thus, the present invention may find application in, for example, the detection of complexes of metals such as strong metal complexes which may be regarded as toxic (e.g. in biological terms when present in the environment).

An example of a metal complex which may be detected in accordance with the present invention is methyl mercury.

By way of further example, entities to be detected may be entities which carry or include analyte species. Thus, for example, entities to be detected may be species formed by interaction of analyte species with a suitable agent or agents.

For example, where analyte species are metal ions the agent or agents may be a complexing agent or complexing agents capable of interacting with the metal ions to form entities (e.g. metal ion complexes) for detection. Thus, for example, metal ions may be formed into complexes of metals by use of a complexing agent or complexing agents and the complexes thus formed may act as entities to be detected in accordance with the present invention.

Thus, for example, metals may be detected in accordance with the present invention.

Examples of complexing agents are chelating agents.

Examples of chelating agents suitable for forming metal chelates are:
ethylene diamine tetra acetate (EDTA), diethylene triamine penta acetate (DTPA), cyclohexylene-dinitrilo tetra acetic acid (CDTA), 1-benzyl-EDTA, derivatives of 1-benzyl-EDTA, 8-hydroxyquinoline, derivatives of 8-hydroxyquinoline, and deferoxamine.

The following are examples of metals which may be analyte species capable of being formed into entities to be detected comprising metal complexes:
calcium (Ca^{II}), iron (Fe^{II}, Fe^{III}), cobalt (Co^{II}, Co^{III}), aluminium (Al^{III}), zinc (Zn^{II}), lead (Pb^{II}), copper (Cu^{II}, Cu^{I}), cadmium (Cd^{II}), vanadium (V^{II}, V^{III}), silver (Ag^{I}, Ag ^{II}), mercury (Hg^{I}, Hg^{II}, indium (In^{III}), manganese (Mn^{II}) and nickel (Ni^{II}).

From the foregoing disclosure, it will be appreciated that in an embodiment of the present invention a method may include the step of forming a complex of an analyte species, said complex thus formed providing an entity to be detected.

Also from the foregoing disclosure, it will be appreciated that in an embodiment of the present invention a method may include the step of linking, either directly or indirectly, an agent to a binding species.

The present invention may find application, for example, in the detection of entities to be detected, in any suitable sample. Thus, for example, samples of water, soil, living species (such as plants (e.g. vegetables) or animals) or air may provide entities to be detected, for detection in accordance with the present invention. By way of example, air-borne species may be entities to be detected and such air-borne species may be, for example, extracted from a sample of air and then subjected to detection. Examples of biological samples in which entities to be detected, may be detected in accordance with the present invention are blood, plasma, serum, urine, saliva and milk. Entities to be detected may be, for example, present in water, an aqueous preparation or a fluid extract (e.g. one prepared by solvent extraction). Entities to be detected may be, for example, haptens.

In accordance with the present invention, by way of example, it is possible to use a support material having a plurality of different auxiliary ligands, each of which auxiliary ligands is provided at a different region of the support material for the detection of different groups of entities.

For example, the same support material, having a given plurality of different auxiliary ligands,may be used (in conjunction with, respectively, binders or auxiliary binders as appropriate to the auxiliary ligand disclosed immediately hereinbefore, to effect separation of a number of different groups of entities.

This is so since the same auxiliary ligands and corresponding binders (e.g. auxiliary binders)may be used to effect selective separation whatever the entities.

This is possible since selectiveness with respect to which entities or groups of entities are to be detected in a given assay may be determined, for example, by the choice of primary species with which a particular binder (e.g. auxiliary binder) may be associated.

It is to be understood that by use of a plurality of auxiliary ligands as immediately hereinbefore disclosed, different entities or different entities associated with detectable species may be selectively separated at different regions of the support material; these regions may then be interrogated separately in a manner which is appropriate to the type of detectable species employed (e.g. optical measurements in the case of a fluorophore) to facilitate detection of a plurality of different entities (each at a different selected region of the support material).

The following are examples of applications in which the present invention may be used in respect of multi-entity detection:
(1) fertility status (male or female) measurements,
(2) fetal health indicators profile measurements,
(3) newborn health status measurements,
(4) thyroid function testing,
(5) high risk infectious microbial disease screening,
(6) autoantibodies detection,
(7) tumour marker detection,
(8) kidney damage marker detection,
(9) screening for drugs of abuse,
(10) screening for pesticides and residues thereof.

It will be appreciated that, for example, a serum protein blocking reagent may, optionally, be used in accordance with the present invention if required for a particular application.

It will also be appreciated that, for example, there may be a need to dilute samples (e.g. with suitable buffer solutions) prior to subjecting to detection.

It is to be understood that the present invention may find application in qualitative detection of an entity or entities, which may also be considered to be qualitative analysis, or in quantitative detection of an entity or entities, which may also be considered to be quantitative analysis (i.e. measurement or determination of an entity or entities).

Also, it will be appreciated that, for example, the present invention may be utilised with samples which contain one or more entities to be detected and with samples which contain substantially no entities to be detected (e.g. such as in the case of a "zero" standard sample or an "unknown" sample, which upon subjecting to detection in accordance with the present invention, is found to contain substantially no entities to be detected). Thus, it will be appreciated that the present invention may be used to "probe" for a plurality of entities, whether or not a particular entity is, or entities are, present in a particular sample.

It will also be appreciated that the present invention may be applied to the detection of a number of different entities over differing concentration ranges.

Further it will be appreciated, by way of example, that by use of standard quantities of authentic entities to be detected, calibration may be effected such that, subsequently, unknown quantities of entities to be detected may be determined.

The present invention may find application in, for example, ratiometric detection (e.g. in ratiometric immunological assay methods) in which more than one detectable species is used.

From the foregoing disclosure it will be appreciated that, for example, where a primary species is associated with a binder (e.g. an auxiliary binder) of an auxiliary ligand-binder pair, and the binder (e.g. auxiliary binder) undergoes binding with the auxiliary ligand provided on a support material, the primary species will become indirectly linked to the support material; it will be appreciated that when such a separation is effected any other species associated with the primary species (e.g. another primary species or a detectable species) will become indirectly linked to the support material.

Thus, by use of a plurality of different auxiliary ligand-binder pairs (e.g. auxiliary ligand-auxiliary binder pairs) and a plurality of different primary species (associated respectively with the auxiliary binder of each auxiliary ligand-auxiliary binder pair) it is possible to effect a plurality of specific separations and thus possible to carry out a plurality of detections for a plurality of entities to be detected.

According to a further aspect of the present invention there is provided apparatus, suitable for use in immunological detection of a plurality of entities, which apparatus includes a support material, or a plurality of support materials, there being provided on the support material, or support materials, when the apparatus is in use, a plurality of different auxiliary ligands, said auxiliary ligands not being primary species, the arrangement being such that, when the apparatus is in use, immunological specific binding interaction between a given auxiliary ligand and a binder therefor facilitates association of a primary species with a support material.

According to a further aspect of the present invention there is provided a test-kit, suitable for use in immunological detection of a plurality of entities, which test-kit includes a support material, or a plurality of support materials, there being provided on the support material, or support materials, when the test-kit is in use, a plurality of different auxiliary ligands, said auxiliary ligands not being primary species, the arrangement being such that, when the test-kit is in use, immunological specific binding interaction between a given auxiliary ligand and a binder therefor facilitates association of a primary species with a support material.

It is to be understood that different auxiliary ligand-binder pairs (e.g. auxiliary ligand-auxiliary binder pairs) of a plurality of such pairs may be chosen such that there is no unacceptable interference between different auxiliary ligand-binder pairs (e.g. auxiliary ligand-auxiliary binder pairs). For example, auxiliary ligand-binder pairs (e.g. auxiliary ligand-auxiliary binder pairs) may be chosen such that there is no degree of interference such as to cause unacceptable interference with the capability of effecting a plurality of specific separations.

By way of example, interference between different auxiliary ligand-binder pairs (e.g. auxiliary ligand-auxiliary binder pairs) may arise if a given binder (e.g. an auxiliary binder) of an auxiliary ligand-binder pair has a tendency to associate with another auxiliary ligand or other auxiliary ligands as well as with the auxiliary ligand with which it is selected to be associated.

Interference may be reduced or substantially avoided by selection of different auxiliary ligand-binder pairs (e.g. auxiliary ligand-auxiliary binder pairs) which are to be utilised in accordance with the present invention.

In summary, various specificities which may be relevant to the capability of carrying out specific separations and multi-entity detection in accordance with the present invention are (I) inter-specificity of different auxiliary ligand-binder pairs (e.g. auxiliary ligand-auxiliary binder pairs) with respect to each other (i.e. the inter-specificity as between different pairs of auxiliary ligands and binders (e.g. auxiliary binders)), (II) the inter-specificity of chosen assays (e.g. with regard to assay reagents), with reference to each other, and (III) the specificity of members of auxiliary ligand-binder pairs (e.g. auxiliary ligand-auxiliary binder pairs) utilised with respect to entities to be detected (and other substances which may be present in a sample).

The specificity to which reference is made at (I) in the immediately preceding paragraph depends upon the level of cross-reactions between different auxiliary ligands and binders (e.g. auxiliary binders) which may be present. Thus, taking the interaction of a given auxiliary ligand and a given binder (e.g. auxiliary binder) of a given pair as 100%, cross reactivities (i.e. interactions of the given auxiliary ligand with binders (e.g. auxiliary binders) other than the binder (e.g. auxiliary binder) of the given pair or the interactions of the given binder (e.g. auxiliary binder) with auxiliary ligands other than the auxiliary ligand of the given pair) preferably should not be greater than 0.01%.

This level of cross-reaction may be achieved, for example, by appropriate choice of auxiliary ligands based upon their chemical structures; thus, for example, maximum practical differences in structures of auxiliary ligands to be used together may lead to a low level of cross-reaction.

Measurement of degrees of cross-reaction may be carried out by standard immunochemical methods.

The specificity to which reference is hereinbefore made at (II) may depend upon cross-reactivities of various entities to be detected with assay reagents (e.g. anti-entity antibodies). This type of specificity may be determined, for example for antibodies, in a manner used for standard antisera currently in use for single entity detection. Development of antisera for use in standard immunoassays involves the measurement of cross-reactions with entity-related substances and with substances known to be present in samples at appreciable concentrations. Thus, such measurements may be applied in accordance with the present invention to determine the specificity of the type to which reference is hereinbefore made in (II).

The specificity to which reference is made hereinbefore in (III) may be arranged to be of a satisfactory level by a suitable choice of auxiliary ligand; thus, for example, a non-biological ligand (i.e. a totally synthetic chemical) may be used. By way of example, measurement of cross-reactions between various entities to be detected and various binders (e.g. auxiliary binders) and of various auxiliary ligands and other assay reagents (e.g. antisera) may be carried out using standard procedures.

It is to be understood that the present invention may, for example, facilitate the use of only one procedure or one set of procedures in the testing of a sample for a plurality of entities.

Thus, for example, in one embodiment of the present invention it is not necessary to perform an assay procedure or a set of assay procedures separately for each entity in a sample. Thus, for example, the present invention may be used in what may be considered to be "simultaneous" detection of a plurality of entities; it will be appreciated that "simultaneous" relates to the performance of a procedure or a set or procedures and not necessarily to various reactions which may occur in a reaction mixture at different speeds and times.

The present invention will now be further described, by way of example only, with reference to a competitive immunoassay method and with reference to a non-competitive immunoassay method.

In the two immunoassay methods described immediately hereinafter, use is made of a plurality of different auxiliary ligand-auxiliary binder species; the auxiliary binder of each pair is an anti-auxiliary ligand antibody. (Where, for example, an auxiliary ligand is a hapten which is antigenic, the anti-auxiliary ligand antibody may be considered to be an anti-auxiliary hapten antibody.) As hereinbefore disclosed auxiliary binders comprising anti-auxiliary ligand antibodies may be prepared by any suitable method, for example those known for the raising of polyclonal and monoclonal antibodies.

By way of example, in a competitive immunoassay method which includes a separation method in accordance with the present invention the following may take place:
(a) a plurality of different auxiliary ligands (of a plurality of different auxiliary ligand-auxiliary binder pairs) are introduced in high concentration to selected regions of a support material (e.g. by covalent linkage) or a support material itself is arranged to provide a plurality of such ligands in selected regions such that the different auxiliary ligands are spatially separated;
(b) a plurality of different auxiliary binders (comprising different antibodies) are linked, by covalent linking, to a plurality of different authentic entities of interest (each of said different auxiliary binders being a binder for one of the auxiliary ligands (i.e. each of the auxiliary binders being a binder for its corresponding auxiliary ligand of a given selected auxiliary ligand-binder pair));
(c) a plurality of different anti-entity antibodies (which may be termed, for example, primary antibodies), being selected such that there is an antibody corresponding to each of the authentic entities, are each labelled with appropriate detectable species capable of giving rise to a signal (e.g. fluorescent substances or enzymes);
(d) in what may be termed primary immune reactions, entities to be detected (if present), suitable amounts of labelled primary antibodies, and authentic entities linked to the auxiliary binders are allowed to mix in solution.

Primary immune reactions may be expected to attain or approach equilibrium in a short period of time (e.g. at a temperature of 37°C to 42°C) thereby to produce a primary immune reaction mixture. It will be appreciated that the primary reaction mixture may contain, *inter alia*, bound and unbound fractions.

Subsequently the primary immune reaction mixture is brought into contact with an "excess" of the plurality of different auxiliary ligands provided on the support material.

(It will be appreciated that "excess" as used in the immediately preceding paragraph means an "excess" in terms of auxiliary ligand sites with respect to the corresponding auxiliary binders.)

Because the plurality of different auxiliary ligands are present in very large excess with respect to the plurality of corresponding auxiliary binders, the binding reactions may be expected to occur rapidly and efficiently with a high proportion of the auxiliary binders becoming bound to the support material. By way of example, in some circumstances, but not all, as much as 99% of the auxiliary binders may become bound to the support material.

Thus, by means of binding between the different auxiliary ligand species and their respective auxiliary binders, some of the primary antibodies carrying detectable species may be retained on the support material by virtue of being linked to appropriate authentic entities which, in turn, are linked to the auxiliary binders. The proportions of retained primary antibodies carrying detectable species will depend upon the concentration of "competing" entities, if any, in a given sample; the competing entity may be a standard or an unknown quantity.

Washing of the support material with appropriate buffers may be used to remove unbound materials and detectable species activity associated with selected regions of the support material may be measured by any suitable method such as those known in the art.

Optionally, detectable species activity may be eluted into solution using appropriate buffers to facilitate measurement of such species.

In some embodiments of the present invention (e.g. embodiments involving the use of an optical immunosensor based on the surface plasmon resonance principle or on evanescent wave fluorescence) a washing step may not be required; thus, detectable species activity associated with the support material (which may be, for example, a sensor surface) may be measured in the presence of unbound material.

It will be understood that, by use of standard quantities of authentic entities, calibration may be effected such that, subsequently, unknown quantities of entities may be determined. It will also be appreciated that, in a competitive immunoassay, as an amount of a given entity in a sample increases, the amount of authentic entity corresponding thereto retained on the support material decreases (and hence the amount of detectable species retained on the support material decreases); it will also be appreciated that, conversely, as the amount of a given entity in a sample decreases, the amount of authentic entity corresponding thereto retained on the support material increases (and hence the amount of detectable species retained on the support material increases), reaching a maximum when no entity is present in a sample.

By way of example, in a non-competitive immunoassay method which involves a separation method in accordance with the present invention the following may be utilised:
(a) a plurality of different anti-entity antibodies [(which may be termed, for example, primary antibodies and which are selected such that there is an antibody corresponding to each entity it is desired to detect) which anti-entity antibodies are to act as "signal" antibodies] which are each conjugated to detectable species which are capable of giving rise to a signal (e.g. a fluorescent substance or an enzyme);
(b) a plurality of different hybrid complexes each of which complexes comprises an "immobilising" antibody (which is also an antibody to an entity to be detected) which is conjugated to an auxiliary binder (e.g. an antibody) for an auxiliary ligand.
(c) a plurality of different entities to be detected (which may be, for example, antigenic substances of high molecular weight);
(d) a support material which itself provides or has attached thereto a plurality of different auxiliary ligands, said different auxiliary ligands being spatially separated.

Also, by way of example, in a non-competitive immunoassay method which includes a separation method in accordance with the present invention the following may take place:
(i) a plurality of different entities of interest and the plurality of "signal" antibodies conjugated to detectable species are mixed in solution whereupon each different entity present is bound by its corresponding signal antibody;
(ii) to the reaction mixture formed in (i) in solution a moderate "excess" of the plurality of hybrid complexes is added whereupon a plurality of different immune complexes are formed in which the entities are "sandwiched";
(iii) the reaction mixture formed in (ii) is brought into contact with an "excess" of the plurality of auxiliary ligands provided on the support material such that binding of the different binders (which binders are conjugated to their respective immobilising antibodies) to the different auxiliary ligands (provided on the support material) takes place. By virtue of these binding reactions, only "signal" antibodies bound to one of the entities to be detected become attached to the support material.

Washing the support material with appropriate buffer may be used to remove unattached "signal" antibodies.

Detectable species left on the support material, the amount of which is directly proportional to the concentration of the entities, may be measured by any suitable method such as those known in the art.

Optionally, detectable species activity may be eluted into solution using appropriate buffers to facilitate measurement of such species.

In some embodiments of the present invention (e.g. embodiments involving the use of an optical immunosensor based on the surface plasmon resonance principle or on evanescent wave fluorescence) a washing step may not be required; thus, detectable species associated with the support material (which may be, for example, a sensor surface) may be measured in the presence of unbound material.

It will be understood that by use of standard quantities of authentic entities calibration may be effected such that, subsequently, unknown quantities of entities may be determined.

It will be appreciated that in heterogenous immunochemical analysis (immunoassay) there is a requirement for the separation of bound and unbound fractions of detectable species in order to permit assessment of the distribution of detectable species between bound and unbound states. It may be considered that this separation step in an immunoassay method is one of the most important features of such a method.

The practical importance of a separation step is based upon the fact that the efficiency of the step, the simplicity (or otherwise) thereof, and the speed thereof may influence the general properties of an assay method and may influence the performance of an assay method.

separation of bound and unbound fractions of detectable species may be effected using a solid phase immobilised antibody technique in which primary antibody is adsorbed onto a surface of a reaction vessel (e.g. polystyrene tubes) or covalently linked to a micro-particulate material such as microcellulose, Sephadex or micro-particulate material with entrapped ferrous oxide (magnetisable particles).

The use of immobilised primary antibodies in separation in an immunoassay may suffer from several disadvantages; for example interaction between primary antibody and an entity (or an authentic entity) may be considerably slower (e.g. due to diffusion considerations) than that which takes place with the same primary antibody and entity (or authentic entity) in solution (i.e. a longer time may be required to reach equilibrium).

Also, the immobilisation of antibody on a solid phase is not a problem-free procedure; thus, for example, it can be difficult to control or reproduce and also serious protein leakage may occur.

The present invention may offer the advantages of reactions in solution with the convenience of a rapid solid-phase separation technique. Thus, for example, a primary immune reaction may be allowed to proceed in solution and then separation may be effected by use of an auxiliary ligand-binder pair (e.g. auxiliary ligand-auxiliary binder pair).

The present invention may also offer for assays and for sensors, for example immunosensors such as optical immunosensors and electrochemical immunosensors, the possibility that a surface carrying auxiliary ligands in accordance with the present invention may be the same for a variety of, or all, entities to be detected thereby providing a generic assay or sensor construction.

A combination comprising a sensor surface carrying auxiliary ligands in accordance with the present invention may also, for example, be such as to permit regeneration for multiple reuse; for example, such a combination of a sensor surface carrying auxiliary ligands may be regenerated by removal of bound substances by washing with a suitable buffer preparation (e.g. 0.1M glycine-HCl at pH 2.5).

Also, by way of further example, auxiliary ligands may be selected so as to be stable to multiple treatment with low or high pH organic solvents and/or chaotropic reagents thereby to permit regeneration for multiple reuse.

The present invention thus offers the possibility of constructing a non-dedicated sensor such as an immunosensor or immunoelectrode.

Thus, by way of example, an electrochemical immunosensor may be provided in which an electrode surface is coated with conducting polymers (e.g. polypyrrole polymer) which may be arranged to act as auxiliary ligands. Thus, for example, different polymers may be used so as to provide different auxiliary ligands. For use in such a sensor antibodies to the conducting polymers (e.g. polypyrrole polymer), or segments thereof, may be generated by suitable techniques such as those known in the art; a combination comprising an electrode surface and coatings of conducting polymers may be, for example, such as to permit regeneration for multiple reuse.

The methods of competitive and non-competitive immunoassay, hereinbefore described by way of example, may be used in an electrochemical immunosensor as immediately hereinbefore described. A detectable species for use in such an electrochemical immunosensor may be arranged to provide an electroactive species.

In conventional electrochemical immunosensors known in the art specific antibodies may be immobilised on an electrode surface thereby to provide a dedicated immunosensor; such immunosensors may have inherent constraints when multiple electrodes with the same properties are required. Thus, disadvantages may become apparent since regeneration of the sensor may be difficult or regeneration may lead to deterioration of antibody activity.

The present invention may offer the possibility of avoiding such disadvantages in that a sensor in accordance with the present invention may not need to be a dedicated sensor.

It will be appreciated from the foregoing that, by way of further example, detection of entities in accordance with the present invention may use an immunosensor technique.

For example, auxiliary ligands may be immobilised on a surface of a sensor device (e.g. on a glass surface, on a quartz surface, or on the surface of an electrode).

For example, in the use of direct optical immunosensors no detectable species is used and the binding of entities to be detected and antibodies may give rise to signal generation to permit detection (e.g. by surface plasmon resonance).

By way of further example, in the use of a detectable species labelled antibodies immunosensor, signal generation may be detected by a suitable method appropriate to the nature of the signal generated by the detectable species (e.g. a means for measuring fluorescent emission may be used when a detectable species is a fluorescence producing species).

By way of example, if desired, the present invention may make use of an inactive species or a plurality of inactive species which may be attached as required to be utilised in accordance with the present invention.

Thus, for example, an inactive species or a plurality of inactive species may be such as to be reconstitutable to an active form.

An inactive form of a species may be prepared by drying and/or freeze-drying (lyophilising).

Thus, for example, any species or combination of species suitable for use in accordance with the present invention may be rendered inactive (e.g. by drying and/or freeze-drying (lyophilising) so as to produce a reconstitutable reagent system or reconstitutable reagent material.

For example, an auxiliary ligand, or a binder for an auxiliary ligand, or an auxiliary binder for an auxiliary ligand, or a primary species, or a plurality of any of these, optionally, in any suitable combination, may be prepared in a reconstitutable form (e.g. by freeze-drying) such that, upon reconstitution, multi-entity analysis may be effected.

Reconstitution may be effected in any suitable manner, for example, by addition of a reconstituting agent such as a suitable solvent (e.g. water), or a suitable solution (e.g. an aqueous solution) or a suitable sample (e.g. an aqueous sample).

The present invention will now be further described, by way of example only, with reference to the accompanying Drawings in which:
- Figure 1: is a diagrammatic representation of an apparatus in accordance with the present invention;
- Figure 2: is a diagrammatic representation of another apparatus in accordance with the present invention;
- Figure 3: is a diagrammatic representation of a further apparatus in accordance with the present invention;
- Figure 4: is a diagrammatic representation of yet a further apparatus in accordance with the present invention; and
- Figure 5: is a diagrammatic representation of a sensor in accordance with the present invention.

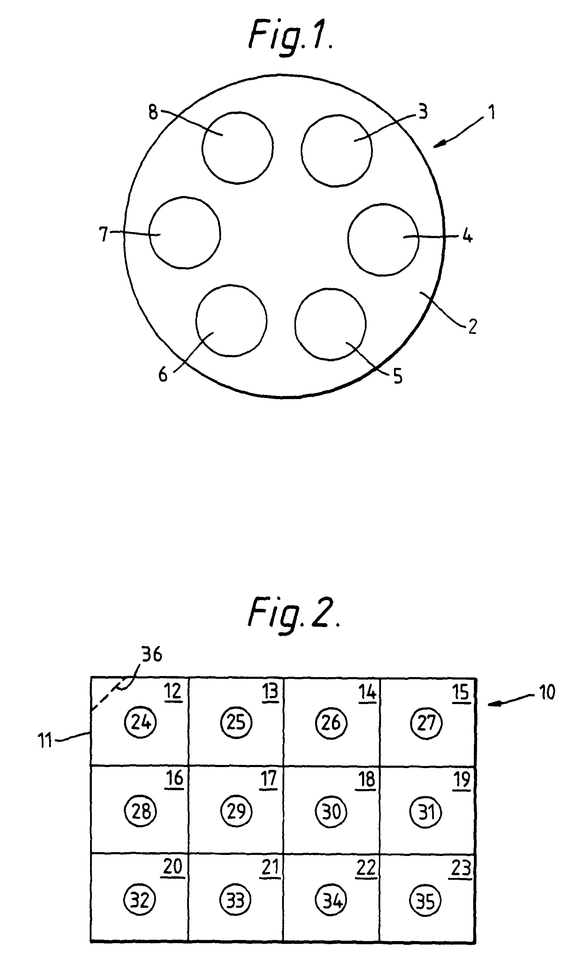

Referring now to Figure 1 of the accompanying Drawings there is shown apparatus 1 having a support material 2 which support material 2 has regions 3, 4, 5, 6, 7 and 8.

In operation the regions 3, 4, 5, 6, 7 and 8 of support material 2 may each provide an auxiliary ligand (not shown). It may be arranged that each of the regions 3, 4, 5, 6, 7 and 8 provides a different auxiliary ligand (i.e. it may be arranged that none of the regions 3, 4, 5, 6, 7 and 8 provides the same auxiliary ligand).

Thus, for example, regions 3, 4, 5, 6, 7 and 8 may respectively provide auxiliary ligands A, B, C, D, E and F (i.e. region 3 may provide auxiliary ligand A, region 4 may provide auxiliary ligand B and so forth).

Upon contacting the apparatus with a material containing a binder (e.g. an auxiliary binder) for auxiliary ligand A, a binder (e.g. an auxiliary binder) for auxiliary ligand B, a binder (e.g. an auxiliary binder) for auxiliary ligand C, a binder (e.g. an auxiliary binder) of auxiliary ligand D, a binder (e.g. an auxiliary binder) for auxiliary ligand E, and a binder (e.g. an auxiliary binder) for auxiliary ligand F, each of the binders (e.g. auxiliary binders) bind specifically, respectively with auxiliary ligands A, B, C, D, E and F.

Thus a plurality of specific separations may be carried out, after which each region will have collected substantially only one type of binder (e.g. one type of auxiliary binder).

Any detectable species associated with each region may then be detected by appropriate interrogation of each region separately. It will be appreciated that the type of interrogation will depend upon the particular detectable species involved (e.g. fluorescence measurements may be made when the detectable species is a fluorophore).

Thus, a sample may be tested for a plurality of different entities (i.e. assays may be carried out for a plurality of different entities using the same support material, and if desired, in the same reaction vessel).

Referring now to Figure 2 of the accompanying Drawings there is shown apparatus 10 having a support material 11, of substantially rectangular plate configuration, having areas 12 to 23 in which of each of areas 12 to 23 there is provided respectively regions 24 to 35.

In operation the regions 24 to 35 of support material 11 may each provide an auxiliary ligand (not shown).

It may be arranged that each of the regions 24 to 35 provides a different auxiliary ligand (i.e. it may be arranged that none of the regions 24 to 35 provides the same auxiliary ligand).

Thus, a plurality of different auxiliary ligands may be provided by the regions 24 to 35 of the support material 11 in a manner similar to that as hereinbefore disclosed with reference to Figure 1 of the accompanying Drawings.

Also a plurality of specific separations may be carried out, and a sample may be tested for a plurality of entities (i.e. an assay may be carried out for a plurality of different entities) in a manner similar to that as hereinbefore disclosed with reference to Figure 1 of the accompanying Drawings.

The support material 11 may be identified in some convenient manner to facilitate the identification of the regions 24 to 35. Thus for example a small portion (e.g. a corner) of the support material 11 may be removed prior to use. For example, a small triangular portion of the outer corner of area 12 may be removed (as indicated by dotted line 36).

The apparatus 10 may be used, for example, in a horizontal position.

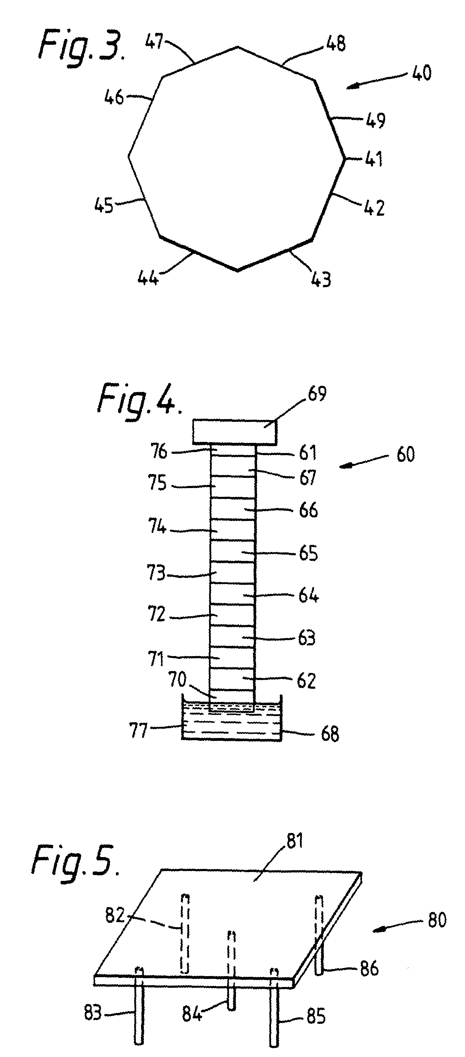

Referring now to Figure 3 of the accompanying Drawings there is shown an apparatus 40. The apparatus 40 has a support material 41 in the form of a regular octahedral prism and thus it will be appreciated that Figure 3 is a plan view of such a prism.

The support material 41 has sides 42 to 49.

In operation regions of the sides 42 to 49 of the support material 41 may each have a region which provides an auxiliary ligand.

It may be arranged that each of the sides 42 to 49 has a region which provides a different auxiliary ligand (i.e. it may be arranged that none of the sides 42 to 49 provides the same auxiliary ligand).

Thus, a plurality of different auxiliary ligands may be provided by the sides 42 to 49 of the support material in a manner similar to that as hereinbefore disclosed with reference to Figure 1 of the accompanying Drawings.

Also a plurality of specific separations may be carried out and a sample may be tested for a plurality of different entities (i.e. assays may be carried out for a plurality or different entities) in a manner similar to that as hereinbefore disclosed with reference to Figure 1 of the accompanying Drawings.

The apparatus 40 may be used, for example, in a vertical position (i.e. with the sides 42 to 49 in a vertical position).

Referring now to Figure 4 of the accompanying Drawings there is shown an apparatus 60 which has a support material 61 in the form of an absorbent wick (e.g. of cellulose paper). The support material 61 has regions 62 to 67.

Also the apparatus 60 has a supply reservoir 68, and a receiving reservoir 69.

In operation the regions 62 to 67 of the support material 61 may each provide an auxiliary ligand (not shown).

Regions 70 to 76 of the support material 61 are such as to provide no auxiliary ligand.

It may be arranged that each of the regions 62 to 67 provides a different auxiliary ligand (i.e. it may be arranged that none of the regions 62 to 67 provides the same auxiliary ligand).

Thus, a plurality of different auxiliary ligands may be provided by regions 62 to 67 of the support material 61 in a manner similar to that as hereinbefore disclosed with reference to Figure 1 or the accompanying Drawings.

Also a plurality of specific separations may be carried out, and a sample may be tested for a plurality of entities (i.e. assays may be carried out for a plurality of different entities) in a manner similar to that as hereinbefore disclosed with reference to Figure 1 of the accompanying Drawings.

Thus, for example, a material (represented as 77) containing a plurality of different binders (e.g. auxiliary binders), may be provided in supply reservoir 68 and the support material 61 may be dipped into the material 77.

As the material 77 rises up the support material 61, by means of capillary action, from supply reservoir 68 to receiving reservoir 69 each different binder (e.g. auxiliary binder) carried by the material will be retained at a different one of the regions 62 to 67 (i.e. a binder (e.g. an auxiliary binder) will be retained at a region which provides its corresponding auxiliary ligand).

Each of the regions 62 to 67 may be interrogated (by a method appropriate to any detectable species present) to detect the presence, absence or amount of different entities.

It will be appreciated that the apparatus 60 may permit sequential separation in accordance with the present invention and thus the regions 62 to 67 may provide a plurality of auxiliary ligands for sequential use.

Referring now to Figure 5 of the accompanying Drawings, there is shown apparatus 80 having a member 81 from which depends a plurality of probes 82, 83, 84, 85 and 86.

(Although only 5 probes are shown, by way of example, the member 81 may carry as many probes as is desired.)

The probes 82 to 86 each comprise support material such that in operation the probes 82 to 86 may each provide regions each of which regions may provide an auxiliary ligand (not shown).

It may be arranged that each of the probes 82 to 86 has a region which provides a different auxiliary ligand (i.e. it may be arranged that none of the probes 82 to 86 provide a region which provides the same auxiliary ligand).

Thus, a plurality of different auxiliary ligands may be provided by the regions of the probes 82 to 86 in a manner similar to that as hereinbefore disclosed with reference to Figure 1 of the accompanying Drawings.

Also, a plurality of specific separations may be carried out and a sample may be tested for a plurality of different entities (i.e. assays may be carried out for a plurality of different entities) in a manner similar to that as hereinbefore disclosed with reference to Figure 1 of the accompanying Drawings.

Thus, for example, a material containing a plurality of different binders (e.g. auxiliary binders) may be contacted with the apparatus 80 such that the material comes into contact with the probes 82 to 86.

Each different binder (e.g. auxiliary binder) in the material will be retained on one of the probes 82 to 86 which provides its corresponding auxiliary ligand.

Subsequently the probes may be individually interrogated for the presence of any detectable species thereon in any suitable manner. Thus, for example, where a detectable species is such as to give rise to an optical signal (e.g. fluorescence) then a probe may be connected to a suitable detector by an optical fibre (not shown) which is connected through the member 81 to an appropriate probe.

By way of further example, where a detectable species is such as to give rise to an electrical signal (e.g. an enzyme capable of generating an electrochemical signal) a probe may be connected to a suitable detector by means of conductors (not shown) which are connected through the member 81 to an appropriate probe.

The present invention will now be further described, by way of example only, as follows.

### Example 1

### Preparation of anti-analyte antisera

17β-estradiol, progesterone and L-thyroxine were selected as entities being analyte species to illustrate, by way of example, the use of a plurality of auxiliary ligand-auxiliary binder pairs in separations in multi-analyte species immunological detection in accordance with the present invention. Accordingly it was necessary to prepare antibodies to the selected entities.

Thus, antiserum to 17β-estradiol was raised in rabbits against 17β-estradiol-3-carboxymethyl-ether-KLH (keyhole limpet haemocyanin), and antiserum to progesterone was raised against progesterone-11-hemisaccinate-KLH and antiserum to L-thyroxine was raised against thyroxine-N-amidosuccinate-KLH in sheep and antibodies obtained in accordance with known procedures.

The antibodies obtained may be considered to be primary antibodies.

### Example 2

### Preparation of antisera to auxiliary ligands

The following were selected, by way of example, to act as auxiliary ligands: 7-hydroxy-4-methyl coumarin-3-propionic acid, 2-(4-aminophenyl)-6-methylthiazole hemiglutarate and 2-phenyl-4-quinoline carboxylic acid. Accordingly it was necessary to prepare antibodies to the selected auxiliary ligands and thus N-hydroxysuccinimide esters of each were prepared by dissolving 2 mmole quantities of each in dimethylformamide (DMF) (25 ml) followed by the addition of 2.1 mmoles of N-hydroxysuccinimide and 2.1 mmoles of dicyclohexylcarbodiimide. The reaction mixtures thus formed were left to stand at 40°C for 3 days.

The resulting three auxiliary ligand active esters were used to prepare an antiserum. Thus, in separate procedures, each of the auxiliary ligand active esters in solution (1 ml) were added slowly to a solution of 50 mg of a mixture of KLH (keyhole limpet haemocyanin) and BSA (bovine serum albumin) dissolved in 3% NaHCO₃ (7 ml, pH 8.6) and dimethylsulphoxide (DMSO) (1 ml).

After mixing for 16 hr, the resulting immunogen solutions were dialysed against 1% NaHCO₃ (4 l) for 3 days to remove unreacted ligand and other low molecular weight substances.

The dialysed immunogen solutions were used separately to raise antisera in animals (rabbits and sheep) in accordance with known procedures.

Antibodies to the auxiliary ligands were obtained from the antisera by means of affinity chromatography using the relevant auxiliary ligands coupled to AH-Sepharose (Registered Trade Mark)-4B (Pharmacia) as affinity matrices.

Thus, each of the three types of antisera were separately subjected to affinity chromatography purification and the antibodies eluted separately by washing each of the matrices with acetonitrile (20%) and propionic acid (1%) in distilled water to give three eluates each one containing antibody to one of the auxiliary ligands. It will be appreciated that the antibody to a particular auxiliary ligand may be considered to be an auxiliary binder.

### Example 3

### conjugating of analyte derivative to the auxiliary binders

The purified antibodies (IgG) (i.e. auxiliary binders) prepared as in Example 2 were conjugated separately to an appropriate analyte species derivative to form three auxiliary binder-authentic entity conjugates (in this Example three auxiliary binder-authentic analyte conjugates).

This was achieved, in separate procedures, by adding active esters of suitable derivatives of 17β-estradiol, progesterone and L-thyroxine (120 nmoles) in dimethylsulphoxide (DMSO) (50 µl) to purified antibody (5 mg), as prepared in Example 2, in 0.1% NaHCO₃ (2.0 ml, pH 8.6) and 0.05M NaCl.

After mixing for 16 hr at 4°C the resulting conjugate from each procedure was purified by gel filtration.

It will be appreciated that the conjugates may be considered to be auxiliary binder-authentic entity conjugates, which in this Example where the entities are analyte species, may be considered to be auxiliary binder-authentic analyte species conjugates.

The conjugates produced in this Example were: sheep anti-[7-hydroxy 4-methyl-coumarin-3-propionic acid]-17β-estradiol-3-carboxybutyryl ether, rabbit anti-[2-(4-aminophenyl)-6-methyl thiazole hemiglutarate]-progesterone-3-carboxy methyloxime, and rabbit anti-[2-phenyl-4-quinoline carboxylic acid]-thyroxine-N-amidoglutarate.

### Example 4

### Providing support materials with auxiliary ligands

Three coating conjugates were prepared by conjugating each of the three auxiliary ligands separately to ovalbumin.

Thus, the appropriate auxiliary active ester in solution (200 µl containing approximately 16 mmoles of auxiliary ligand active ester (see Example 2)) was added to ovalbumin (25 mg) in 3% NaHCO₃ (5 ml, pH 8.6) and DMSO (1 ml).

The resulting reaction mixture was stirred for 16 hr at room temperature before being dialysed against 1% NaHCO₃ for 3 days and subsequently treated with Norrit A activated charcoal (0.3%).

By carrying out the conjugation as immediately hereinbefore disclosed for each of the three auxiliary ligands three purified solutions were obtained, one containing a conjugate of ovalbumin and 7-hydroxy-4-methyl coumarin-3-propionic acid, one containing a conjugate of ovalbumin and 2-(4-aminophenyl)-6-methyl thiazole hemiglutarate, and one containing a conjugate of ovalbumin and 2-phenyl-4-quinoline carboxylic acid.

The auxiliary ligand-ovalbumin conjugates were used for adsorbing auxiliary ligands to a support material by coating polystyrene microtitre plates.

Thus, one conjugate was used to introduce 7-hydroxy-4-methyl coumarin-3-propionic acid to a support material, another conjugate was used to introduce 2-(4-aminophenyl)-6-methyl thiazole hemiglutarate to a support material, and the other conjugate was used to introduce 2-phenyl-4-quinoline carboxylic acid to a support material.

Plates were coated by exposing them to a chosen auxiliary ligand-ovalbumin conjugate. The coating was carried out by use of a solution containing 4 µg/ml of a chosen conjugate in 0.05M NaHCO₃.

Thus, 200 µl of solution per well was used and left at 4°C for 24 hrs. Excess sites were subsequently blocked by horse haemoglobin (0.5 mg/ml).

### Example 5

### Multianalyte species immunoassay

In this Example three entities being analyte species were used and thus this Example illustrates multianalyte species immunoassay.

A range of standard solutions was prepared each containing a mixture of analyte species standards.

The concentrations of the various analyte species for each standard solution are shown below in Table I:

**Table I**

| Standard Solution | A | B | C | D |
|---|---|---|---|---|
| Estradiol (pg/100 µl) | 12.5 | 25 | 50 | 100 |
| Progesterone (pg/100 µl) | 50 | 100 | 200 | 400 |
| Thyroxine (pg/100 µl) | 100 | 200 | 400 | 800 |

The analyte species standards were made up in 10mM phosphate (pH 7.5) containing 0.1M NaCl, 50 µg/ml rhodamine base and 1 mg/ml ovalbumin.

Also a 'standard' solution containing no analyte species was used.

In order to carry out immunoassay for the analyte species, in the Example an ELISA (enzyme labelled immunosorbent assay), 100 µl volumes of the standard solutions A to D, and of the standard containing no analyte species, were added in duplicate to wells of microtitre ELISA plates.

A mixture of anti-analyte species antibodies, in a suitable buffer solution, (said antibodies being primary antibodies as prepared in Example 1) was added (50 µl) to the wells.

After 30 min at 37°C, 50 µl volumes of a mixture of auxiliary binder-authentic analyte species, in a suitable buffer solution, were added to the wells and the assay allowed to continue for a further 15 minutes.

In order to effect separation in accordance with the present invention 180 µl volumes of assay mixtures from the wells were first transferred to a microtitre plate coated with the auxiliary ligand 7-hydroxy-4-methyl coumarin-3-propionic acid (see Example 1) and left for 10 minutes so as selectively to immobilize bound fraction of the estradiol assay.

Subsequently, 150 µl volumes of the assay mixtures were transferred to a microtitre plate coated with the auxiliary ligand 2-phenyl-4-quinoline carboxylic acid (see Example 4) and left for 10 minutes so as selectively to immobilise bound fraction of the thyroxine assay.

Subsequently, 120 µl volume of the assay mixtures were transferred to a microtitre plate coated with the auxiliary ligand 2-(4-amminophenyl)-6-methyl thiazole hemiglutarate (see Example 4) and left for 10 minutes so as selectively to immobilise bound fraction of the progesterone assay.

The plates retaining bound fractions of the three analyte species were washed and appropriate commercially available second antibody-horse radish peroxidase conjugates were added. The second antibody-horse radish peroxidase conjugate used for estradiol assay had an anti-rabbit immunoglobulin antibody and the second antibody-horse radish peroxidase conjugate used for progesterone and thyroxine assays had an anti-sheep immunoglobulin antibody.

The plates were then developed in accordance with known procedures using ABTS and hydrogen peroxide and Optical Densities (O.D.) values were read at 405 nm.

The results are given in Tables II, III and IV.

**Table II**

| Concentration 17β Estradiol (pg/well) | O.D. at 405 nm (Average of 2 readings) |
|---|---|
| 0 | 1.23 |
| 12.5 | 1.11 |
| 25 | 0.81 |
| 50 | 0.62 |
| 100 | 0.48 |

**Table III**

| Concentration Progesterone (pg/well) | O.D. at 405 nm (Average of 2 readings) |
|---|---|
| 0 | 1.41 |
| 50 | 0.92 |
| 100 | 0.75 |
| 200 | 0.50 |
| 400 | 0.31 |

**Table IV**

| Concentration Thyroxine (pg/well) | O.D. at 405 nm (Average of 2 readings) |
|---|---|
| 0 | 1.90 |
| 100 | 1.80 |
| 200 | 1.60 |
| 400 | 1.00 |
| 800 | 0.35 |

## Claims

1. A separation method **characterised in that** said separation method is suitable for use in an immunological method for the detection of a plurality of entities and **in that** said separation method includes using a plurality of different auxiliary ligand-binder pairs, the auxiliary ligand of each of the plurality of auxiliary ligand-binder pairs being provided on a support material, said auxiliary ligands not being primary species, said separation method being such that immunological specific binding interaction between a given auxiliary ligand and a binder therefor facilitates association of a primary species with a support material.

2. A separation method as claimed in Claim 1 further **characterised in that** said separation method includes using a plurality of different auxiliary ligand-auxiliary binder pairs, the auxiliary ligands and the auxiliary binders of the pairs not being primary species, and the auxiliary ligand of each of the plurality of auxiliary ligand-auxiliary binder pairs being provided on a support material.

3. A method **characterised in that** said method is suitable for use in immunological detection of a plurality of entities and **in that** said method includes using a plurality of different auxiliary ligand-binder pairs, the auxiliary ligand of each of the plurality of auxiliary ligand-binder pairs being provided on a support material, said auxiliary ligands not being primary species, said method being such that immunological specific binding interaction between a given auxiliary ligand and a binder therefor facilitates association of a primary species with a support material.

4. A method as claimed in Claim 3 further **characterised in that** said method includes using a plurality of different auxiliary ligand-auxiliary binder pairs, the auxiliary ligands and the auxiliary binders of the pairs not being primary species, and the auxiliary ligand of each of the plurality of auxiliary ligand-auxiliary binder pairs being provided on a support material.

5. A sensor **characterised in that** said sensor is suitable for use in immunological detection of a plurality of entities and **in that** said sensor includes a support material there being provided on the support material when the sensor is in use a plurality of different auxiliary ligands, said auxiliary ligands not being primary species, the arrangement being such that, when the sensor is in use, immunological specific binding interaction between a given auxiliary ligand and a binder therefor facilitates association of a primary species with the support material.

6. A method or a sensor as claimed in any one of the preceding Claims further **characterised in that** an auxiliary ligand is 2,4 dinitrophenol, fluorescein, digitoxin, coumarin, cibacron blue (Registered Trade Mark), 2-(4-aminophenyl)-6-methyl benzothiazole-hemiglutarate, camphorcarboxylic acid, 4-amino-benzo-15-crown-5, carboxyfluorescein, 3-methyl-1-adamantane acetic acid, 2-phenyl-4-quinoline carboxylic acid, xanthine-9-carboxyamide-glycine-glycine, 4-hydroxy-7-trifluoromethyl-3-quinaldine carboxylic acid, cis-bicyclo [3,3,0] octane-2-carboxylic acid, endo-bicyclo [2,2,2] oct-5-ene-2,3-dicarboxylic anhydride, N-[4-(4-aminobenzyl) phenyl]-5-norbornene-2,3-dicarboximide, or [IR-(2-endo, 3-exo)]-3-hydroxy-4,7,7-trimethyl bicyclo [2,2,1] heptane-2-acetic acid.

7. A method or a sensor as claimed in any one of Claims 1 to 5 further **characterised in that** an auxiliary ligand is provided as a coating of a polymer on a support material.

8. A method or a sensor as claimed in any one of Claims 1 to 7 further **characterised in that** the binder is an auxiliary binder and the auxiliary binder is anti-2,4 dinitrophenol antibody, anti-fluorescein antibody, anti-digitoxin antibody, anti-coumarin antibody, anti-cibacron blue antibody, anti-2-(4-aminophenyl)-6-methyl benzothiazole-hemiglutarate antibody, anti-camphorcarboxylic acid antibody, anti-4-amino-benzo-15-crown-5 antibody, anti-carboxyfluorescein antibody, anti-3-methyl-1-adamantane acetic acid antibody, anti-2-phenyl-4-quinoline carboxylic acid antibody, anti-xanthine-9-carboxyamide-glycine-glycine antibody, anti-4-hydroxy-7-trifluoromethyl-3-quinaldine carboxylic acid antibody, anti-cis-bicyclo [3,3,0] octane-2-carboxylic acid antibody, anti-endo-bicyclo [2,2,2] oct-5-ene-2,3-dicarboxylic anhydride antibody, anti-N-[4-(4-aminobenzyl) phenyl]-5-norbornene-2,3-dicarboximide antibody, or anti-[IR-(2-endo, 3-exo)]-3-hydroxy-4,7,7-trimethyl bicyclo [2,2,1] heptane-2-acetic acid antibody.

9. A method or sensor as claimed in any one of Claims 1 to 5 further **characterised in that** a binder is a species which has a part which may provide an auxiliary function.

10. A method as claimed in Claim 9 further **characterised in that** a binder is a bi-functional antibody.

11. A method or a sensor as claimed in any one of the preceding claims further **characterised in that** different regions or zones of a support material are arranged to provide different auxiliary ligands, such that different specific separations may be effected on different regions or zones of the support material.

12. A method or a sensor as claimed in any one of the preceding Claims further **characterised in that** the support material itself, or a part of the support material itself, provides an auxiliary ligand.

13. A method or a sensor as claimed in any one of Claims 1 to 11 further **characterised in that** an auxiliary ligand is attached to a support material.

14. A method or a sensor as claimed in any one of Claims 1 to 11 or claim 13 further **characterised in that** an auxiliary ligand is directly attached to a support material.

15. A method or a sensor as claimed in any one of Claims 1 to 11 or Claim 13 further **characterised in that** an auxiliary ligand is indirectly attached to a support material.

16. A method or a sensor as claimed in any one of Claims 1 to 11 further **characterised in that** an oligomer of an auxiliary ligand, or a polymer of an auxiliary ligand is attached directly or indirectly to a support material.

17. A method or a sensor as claimed in any one of the preceding Claims further **characterised in that** a support material is a reaction vessel wall, an insoluble polysaccharide, a microparticle, polystyrene, cross-linked dextran, an insoluble polymer structure, a glass surface, a derivatised silica surface, a polymer attached to a surface, a microparticulate material with entrapped ferrous oxide, nylon or a polyamide.

18. A method as claimed in any one of any one of the preceding Claims further **characterised in that** said method also includes the step of attaching, either directly, or indirectly, an auxiliary ligand to a support material.

19. A method or a sensor as claimed in any one of Claims 1 to 16, or Claim 18 further **characterised in that** a support material is in the form of a carrier which may be moved from a sample application means, for applying a sample to the carrier, to a detection means thereby to allow successive detection of a plurality of different entities.

20. A method or a sensor as claimed in any one of the preceding Claims further **characterised in that** an auxiliary ligand or an auxiliary binder is associated with a primary species and the primary species is a primary antibody.

21. A method or a sensor as claimed in any one of Claims 1 to 19 further **characterised in that** an auxiliary ligand or an auxiliary binder is associated with a primary species and the primary species is a ligand.

22. An immunological detection or immunoassay **characterised in that** said immunological detection method includes a separation method as claimed in Claim 1 or Claim 2.

23. A method as claimed in any one of Claims 3, 4, or 6 to 19 further **characterised in that** said method includes immunological detection or immunoassay comprising heterogenous immunoassay.

24. A method as claimed in any one of Claims 3, 4 or 6 to 19 further **characterised in that** said method includes immunological detection or immunoassay comprising homogeneous immunoassay.

25. A method or a sensor as claimed in any one of the preceding claims further **characterised by** including a detectable species.

26. A method or a sensor as claimed in claim 25 further **characterised in that** a detectable species is an enzyme, a species capable of giving a fluorescent signal, a chemiluminescent compound, a bioluminescent compound, a dye, or a radioisotope.

27. A method or a sensor as claimed in Claim 25 further **characterised in that** a detectable species is a ligand species, a polymer of a ligand species, a binder species, or a polymer of a binder species.

28. A method or a sensor as claimed in claim 27 further **characterised in that** a ligand species may be detected by a binder species associated with a tracer species.

29. A method or a sensor as claimed in Claim 27 further **characterised in that** a binder species may be detected by a ligand species associated with a tracer species.

30. A method or a sensor as claimed in Claim 28 or Claim 29 further **characterised in that** the tracer species is an enzyme.

31. A method or a sensor as claimed in Claim 28 or Claim 29 further **characterised in that** the tracer species is a species capable of giving a fluorescent signal, a chemiluminescent compound, a bioluminescent compound, a radioisotope or a dye.

32. A method or a sensor as claimed in Claim 28 further **characterised in that** the ligand species is an antigenic species.

33. A method or a sensor as claimed in Claim 28 further **characterised in that** the ligand species is a non-antigenic ligand species.

34. A method or a sensor as claimed in Claim 29 further **characterised in that** the binder species is an antibody.

35. A method or a sensor as claimed in Claim 29 further **characterised in that** the binder species is a binder species of a non-antigenic ligand species.

36. A method or a sensor as claimed in any one of the preceding Claims further **characterised in that** entities to be detected are themselves analyte species.

37. A method or a sensor as claimed in any one of Claims 1 to 35 further **characterised in that** entities to be detected carry or include analyte species.

38. A method or a sensor as claimed in claim 36 further **characterised in that** the entities to be detected are steroid hormones, thyroid hormones, steroids in extracts, drugs, polypeptide hormones, tumour markers, protein antigens, blood proteins, marker proteins, pesticides, toxins, micro-organisms, antibodies to micro-organisms, or metal complexes.

39. A method or a sensor as claimed in claim 37 further **characterised in that** analyte species are metal ions.

40. A method as claimed in any one of the preceding claims further **characterised in that** a reconstitutable reagent system or a reconstitutable reagent material is used.

41. Apparatus **characterised in that** said apparatus is suitable for use in immunological detection of a plurality of entities and **in that** said apparatus includes a support material, or a plurality of support materials, there being provided on the support material, or support materials, when the apparatus is in use, a plurality of different auxiliary ligands, said auxiliary ligands not being primary species, the arrangement being such that, when the apparatus is in use, immunological specific binding interaction between a given auxiliary ligand and a binder therefor facilitates association of a primary species with a support material.

42. A test-kit **characterised in that** said test-kit is suitable for use in immunological detection of a plurality of entities and **in that** said test-kit includes a support material, or a plurality of support materials, there being provided on the support material, or suppport materials, when the test-kit is in use, a plurality of different auxiliary ligands, said auxiliary ligands not being primary species, the arrangement being such that, when the test-kit is in use, immunological specific binding interaction between a given auxiliary ligand and a binder therefor facilitates association of a primary species with a support material.

## Patentansprüche

1. Trennunssverfahren, **dadurch gekennzeichnet, daß** das Trennungsverfahren zur Verwendung in einem immunologischen Verfahren zum Nachweis einer Vielzahl von einzelnen Stoffen geeignet ist und daß das Trennungsverfahren die Verwendung einer Vielzahl von unterschiedlichen Hilfsligand-Binder Paaren umfaßt, wobei der Hilfsligand jeder der Vielzahl von Hilfsligand-Binder Paaren auf einem Trägermaterial vorgesehen ist, die Hilfsliganden keine Primärspezien sind und das Trennungsverfahren so ist, daß eine immunologische, spezifische Bindungswechselwirkung zwischen einem gegebenen Hilfsliganden und einem Binder dafür die Assoziation einer Primärspezies mit einem Trägermaterial erleichtert.

2. Trennungsverfahren nach Anspruch 1, weiterhin **dadurch gekennzeichnet, daß** das Trennungsverfahren die Verwendung einer Vielzahl von unterschiedlichen Hilfsligand-Hilfsbinder Paaren umfaßt, wobei die Hilfsliganden und die Hilfsbinder der Paare keine Primärspezien sind und der Hilfsligand jeder der Vielzahl von Hilfsligand-Hilfsbinder Paaren auf einem Trägermaterial vorgesehen ist.

3. Verfahren, **dadurch gekennzeichnet, daß** das Verfahren zur Vervendung beim immunologischen Nachweis einer Vielzahl von einzelnen Stoffen geeignet ist und daß das Verfahren die Vervendung einer Vielzahl von unterschiedlichen, Hilfsligand-Binder Paaren umfaßt, wobei der Hilfsligand jeder der Vielzahl von Hilfsligand-Binder Paaren auf einem Trägermaterial vorgesehen ist, die Hilfsliganden keine Primärspezien sind und das Verfahren so ist, daß eine immunologische, spezifische Bindungswechselwirkung zwischen einem gegebenen Hilfsliganden und einem Binder dafür die Assoziation einer Primär-spezies mit einem Trägermaterial erleichtert.

4. Verfahren nach Anspruch 3, weiterhin **dadurch gekennzeichnet, daß** das Verfahren die Vervendung einer Vielzahl von unterschiedlichen Hilfsligand-Hilfsbinder Paaren umfaßt, wobei die Hilfsliganden und Hilfsbinder der Paare keine Primärspezien sind und der Hilfsligand der Vielzahl von Hilfsligand-Hilfsbinder Paaren auf einem Trägermaterial vorgesehen ist.

5. Sensor, **dadurch gekennzeichnet, daß** der Sensor zur Verwendung beim immunologischen Nachweis einer Vielzahl von einzelnen Stoffen geeignet ist und daß der Sensor ein Trägermaterial umfaßt, wobei dort auf dem Trägermaterial eine Vielzahl von unterschiedlichen Hilfsliganden vorgesehen ist, wenn der Sensor in Gebrauch ist, die Hilfsliganden keine Primärspezien sind und die Anordnung so ist, daß, wenn der Sensor in Gebrauch ist, eine immunologische, spezifische Bindungswechselwirkung zwischen einem gegebenen Hilfsliganden und einem Binder dafür die Assoziation einer Primärspezies mit dem Trägermaterial erleichtert.

6. Verfahren oder Sensor nach einem der vorausgehenden Ansprüche, weiterhin **dadurch gekennzeichnet, daß** ein Hilfsligand 2,4-Dinitrophenol, Fluorescein, Digitoxin, Cumarin, Cibacron Blau (eingetragene Marke), 2-(4-Aminophenyl)-6-methyl-benzothiazol-hemigluterat, Camphercarbonsäure, 4-Amino-benzo-15-krone-5, Carboxyfluorescein, 3-Methyl-1-adamantan-essigsäure, 2-Phenyl-4-chinolin-carbonsäure, Xanthin-9-carboxyamid-glycinglycin, 4-Hydroxy-7-trifluormethyl-3-chinaldin-carbonsäure, cis-Bicyclo [3,3,0] octan-2-carbonsäure, endo-Bicyclo [2,2,2] oct-5-en-2,3-dicarbonsäureanhydrid, N-[4-(4-aminobenzyl)phenyl]-5-norbornen-2,3-dicarboximid oder [IR-(2-endo, 3-exo)]-3-hydroxy-4, 7,7-trimethyl-bicyclo [2,2,1] heptan-2-essigsäure ist.

7. Verfahren oder Sensor nach einem der Ansprüche 1 bis 5, weiterhin **dadurch gekennzeichnet, daß** ein Hilfsligand vorgesehen ist als eine Beschichtung eines Polymers auf einem Trägermaterial.

8. Verfahren oder Sensor nach einem der Ansprüche 1 bis 7, weiterhin **dadurch gekennzeichnet, daß** der Binder ein Hilfsbinder ist und der Hilfsbinder ein Anti-2,4-Dinitrophenol Antikörper, Anti-Fluorescein Antikörper, Anti-Digitoxin Antikörper, Anti-Cumarin Antikörper, Anti-Cibacron Blau Antikörper, Anti-2-(4-Aminophenyl)-6-methyl-benzothiazol-hemiglutarat Antikörper, Anti-Camphercarbonsäure Antikörper, Anti-4-Aminobenzo-15-krone-5 Antikörper, Anti-Carboxyfluorescein Antikörper, Anti-3-Methyl-1-adamantan-essigsäure Antikörper, Anti-2-Phenyl-4-chinolin-carbonsäure Antikörper, Anti-Xanthin-9-carboxyamid-glycin-glycin Antikörper, Anti-4-Hydroxy-7-trifluormethyl-3-chinaldin-carbonsäure Antikörper, Anti-cis-Bicyclo [3,3,0]octan-2-carbonsäure Antikörper, Anti-endo-Bicyclo [2,2,2]oct-5-en-2,3-dicarbonsäureanhydrid Antikörper, Anti-N-[4-(4-Aminobenzyl)phenyl]-5-norbornen-2,3-dicarboximid Antikörper oder Anti-[IR-(2-endo, 3-exo)]-3-Hydroxy-4,7,7-trimethyl-bicyclo[2,2,1]heptan-2-essigsäure Antikörper ist.

9. Verfahren oder Sensor nach einem der Ansprüche 1 bis 5, weiterhin **dadurch gekennzeichnet, daß** ein Binder eine Spezies ist, die einen Teil aufweist, der eine Hilfsfunktion liefern kann.

10. Verfahren nach Anspruch 9, weiterhin **dadurch gekennzeichnet, daß** ein Binder ein bifunktioneller Antikörper ist.

11. Verfahren oder Sensor nach einem der vorausgehenden Ansprüche, weiterhin **dadurch gekennzeichnet, daß** unterschiedliche Regionen oder Zonen eines Trägermaterials angeordnet sind, um unterschiedliche Hilfsliganden vorzusehen, so daß unterschiedliche, spezifische Trennungen auf unterschiedlichen Regionen oder Zonen des Trägermaterials bewirkt werden können.

12. Verfahren oder Sensor nach einem der vorausgehenden Ansprüche, weiterhin **dadurch gekennzeichnet, daß** das Trägermaterial selbst oder ein Teil des Trägermaterials selbst einen Hilfsliganden darstellt.

13. Verfahren oder Sensor nach einem der Ansprüche 1 bis 11, weiterhin **dadurch gekennzeichnet, daß** ein Hilfsligand an ein Trägermaterial gebunden ist.

14. Verfahren oder Sensor nach einem der Ansprüche 1 bis 11 oder Anspruch 13, weiterhin **dadurch gekennzeichnet, daß** ein Hilfsligand direkt an ein Trägermaterial gebunden ist.

15. Verfahren oder Sensor nach einem der Ansprüche 1 bis 11 oder Anspruch 13, weiterhin **dadurch gekennzeichnet, daß** ein Hilfsligand indirekt an ein Trägermaterial gebunden ist.

16. Verfahren oder Sensor nach einem der Ansprüche 1 bis 11, weiterhin **dadurch gekennzeichnet, daß** ein Oligomer eines Hilfsliganden oder ein Polymer eines Hilfsliganden direkt oder indirekt an ein Trägermaterial gebunden ist,

17. Verfahren oder Sensor nach einem der vorausgehenden Ansprüche, weiterhin **dadurch gekennzeichnet, daß** ein Trägermaterial eine Reaktionsgefäßwand, ein unlösliches Polysaccharid, ein Mikroteilchen, Polystyrol, vernetztes Dextran, eine unlösliche Polymerstruktur, eine Glasoberfläche, eine derivatisierte Silicaoberfläche, ein an eine Oberfläche gebundenes Polymer, ein Mikropartikelmaterial mit eingeschlossenem Eisen(II)-oxid, Nylon oder ein Polyamid ist.

18. Verfahren nach einem der vorausgehenden Ansprüche, weiterhin **dadurch gekennzeichnet, daß** das Verfahren den Schritt des Verbindens, entweder direkt oder indirekt, eines Hilfsliganden an ein Trägermaterial umfaßt.

19. Verfahren oder Sensor nach einem der Ansprüche 1 bis 16 oder Anspruch 18, weiterhin **dadurch gekennzeichnet, daß** ein Trägermaterial in Form eines Carriers vorliegt, der von einem Probenaufbringmittel, um eine Probe auf den Carrier aufzubringen, zu einem Nachweismittel bewegt werden kann, um dadurch den aufeinanderfolgenden Nachweis einer Vielzahl von unterschiedlichen einzelnen Stoffen zu gestatten.

20. Verfahren oder Sensor nach einem der vorausgehenden Ansprüche, weiterhin **dadurch gekennzeichnet, daß** ein Hilfsligand oder ein Hilfsbinder mit einer Primärspezies assoziiert ist und die Primärspezies ein Primärantikörper ist.

21. Verfahren oder Sensor nach einem der Ansprüche 1 bis 19, weiterhin **dadurch gekennzeichnet, daß** ein Hilfsligand oder ein Hilfsbinder mit einer Primärspezies assoziiert ist und die Primärspezies ain Ligand ist.

22. Immunologischer Nachweis oder Immunoassay, **dadurch gekennzeichnet, daß** das immunologische Nachweisverfahren ein Trennungsverfahren nach Anspruch 1 oder Anspruch 2 umfaßt.

23. Verfahren nach einem der Ansprüche 3, 4 oder 6 bis 19, weiterhin **dadurch gekennzeichnet, daß** das Verfahren einen immunologischen Nachweis oder Immunoassay einschließt, das ein heterogenes Immunoassay umfaßt

24. Verfahren nach einem der Ansprüche 3, 4 oder 6 bis 19, weiterhin **dadurch gekennzeichnet, daß** das Verfahren einen immunologischen Nachweis oder Immunoassay einschließt, des ein homogenes Immunoassay umfaßt.

25. Verfahren oder Sensor nach einem der vorausgehenden Ansprüche, weiterhin **dadurch gekennzeichnet, daß** es eine nachweisbare Spezies umfaßt.

26. Verfahren oder Sensor nach Anspruch 25, weiterhin **dadurch gekennzeichnet, daß** eine nachweisbare Spezies ein Enzym, eine Spezies, die ein fluoreszierendes Signal geben kann, eine chemolumineszierende Verbindung, eine biolumineszierende Verbindung, ein Farbstoff oder ein Radioisotop ist.

27. Verfahren oder Sensor nach Anspruch 25, weiterhin **dadurch gekennzeichnet, daß** eine nachweisbare Spezies eine Ligandspezies, ein Polymer einer Ligandspezies, eine Binderspezies oder ein Polymer einer Binderspezies ist.

28. Verfahren oder Sensor nach Anspruch 27, weiterhin **dadurch gekennzeichnet, daß** eine Ligandspezies durch eine Binderspezies, die mit einer Tracerspezies assoziiert ist, nachgewiesen werden kann.

29. Verfahren oder Sensor nach Anspruch 27, weiterhin **dadurch gekennzeichnet, daß** eine Binderspezies durch eine Ligendspezies, die mit einer Tracerspezies assoziiert ist, nachgewiesen werden kann.

30. Verfahren oder Seasor nach Anspruch 28 oder Anspruch 29, weiterhin **dadurch gekennzeichnet, daß** die Tracerspezies ein Enzym ist.

31. Verfahren oder Sensor nach Anspruch 28 oder Anspruch 29, weiterhin **dadurch gekennzeichnet, daß** die Tracerspezies eine Spezies, die ein fluoreszierendes Signal abgeben kann, eine chemolumineszierende Verbindung, eine biolumineszierende Verbindung, ein Radioisotop oder ein Farbstoff ist.

32. Verfahren oder Sensor nach Anspruch 28, weiterhin **dadurch gekennzeichnet, daß** die Ligandspezies eine antigene Spezies ist.

33. Verfahren oder Sensor nach Anspruch 28, weiterhin **dadurch gekennzeichnet, daß** die Ligandspezies eine nicht-antigene Ligandspezies ist.

34. Verfahren oder Sensor nach Anspruch 29, weiterhin **dadurch gekennzeichnet, daß** die Binderspezies ein Antikörper ist.

35. Verfahren oder Sensor nach Anspruch 29, weiterhin **dadurch gekennzeichnet, daß** die Biodergpezies eine Binderspezies einer nicht-antigenen Ligandspezies ist.

36. Verfahren oder Sensor nach einem der vorausgehenden Ansprüche, weiterhin **dadurch gekennzeichnet, daß** die nachzuweisenden einzelnen Stoffe selbst eine Analytspezies sind.

37. Verfahren oder Sensor nach einem der Ansprüche 1 bis 35, weiterhin **dadurch gekennzeichnet, daß** die nachzuweisenden einzelnen Stoffe Analytspezien tragen oder einschließen.

38. Verfahren oder Sensor nach Anspruch 36, weiterhin **dadurch gekennzeichnet, daß** die nachzuweisenden einzelnen Stoffe Steroid-Hormone, Thyroid-Hormone, Steroide in Extrakten, Arzneimittel, Polypeptid-Hormone, Tumormarker, Protein-Antigene, Blutproteine, Markerproteine, Pestizide, Toxine, Mikroorganismen, Antikörper für Mikroorganismen oder Metallkomplexe sind.

39. Verfahren oder Sensor nach Anspruch 37, weiterhin **dadurch gekennzeichnet, daß** die Analytspezien Metallionen sind.

40. Verfahren nach einem der vorausgehendan Ansprüche, weicerhin **dadurch gekennzeichnet, daß** ein rückbildbares Reagenssystem oder ein rückbildbares Reagensmaterial verwendet wird.

41. Vorrichtung, **dadurch gekennzeichnet, daß** die Vorrichtung zur Verwendung beim immunologischen Nachweis einer Vielzahl von einzelnen Stoffen geeignet ist und daß die Vorrichtung ein Trägermaterial oder eine Vielzahl von Trägermaterialien umfaßt, wobei dort auf dem Trägermaterial oder den Trägermaterialien eine Vielzahl von unterschiedlichen Hilfsliganden vorgesehen ist, wenn die Vorrichtung in Gebrauch ist, wobei die Hilfsliganden keine Primärspezien sind und die Anordnung so ist, daß, wenn die Vorrichtung in Gebrauch ist, eine immunologische, spezifische Bindungswechselwirkung zwischen einem gegebenen Hilfsliganden und einem Binder dafür die Assoziation einer Primärspezies mit einem Trägermaterial erleichtert.

42. Testkit, **dadurch gekennzeichnet, daß** das Testkit für die Verwendung beim immunologischen Nachweis einer Vielzahl von einzelnen Stoffen geeignet ist und daß des Testkit ein Trägermaterial oder eine Vielzahl von Trägermaterialien umfaßt, wobei dort auf dem Trägermaterial oder den Trägermaterialien eine Vielzahl von unterschiedlichen Hilfsliganden vorgesehen ist, wenn das Testkit in Gebrauch ist, die Hilfsliganden keine Primärspezien sind und die Anordnung so ist, daß, wenn das Testkit in Gebrauch ist, eine immunologische, spezifische Wechselwirkung zwischen einem gegebenen Hilfsliganden und einem Binder dafür die Assoziation zwischen einer Primärspezies mit einem Trägermaterial erleichtert.

## Revendications

1. Procédé de séparation, **caractérisé en ce que** ledit procédé de séparation est utilisable dans un procédé immunologique pour la détection d'une pluralité d'entités et **en ce que** ledit procédé de séparation comprend l'utilisation d'une pluralité de paires liant-ligand auxiliaire différentes, le ligand auxiliaire de chacune des pluralités de paires liant-ligand auxiliaire étant fourni sur un matériau support, lesdits ligands auxiliaires n'étant pas des espèces primaires, ledit procédé de séparation étant tel qu'une interaction de liaison spécifique immunologique entre un ligand auxiliaire donné et un liant correspondant facilite l'association d'une première espèce avec un matériau support.

2. Procédé de séparation selon la revendication 1, **caractérisé en outre en ce que** ledit procédé de séparation comprend l'utilisation d'une pluralité de paires liant auxiliaire-ligand auxiliaire différentes, les ligands auxiliaires et les liants auxiliaires des paires n'étant pas des espèces primaires, et le ligand auxiliaire de chacune des pluralités de paires liant auxiliaire-ligand auxiliaire étant fourni sur un matériau support.

3. Procédé **caractérisé en ce que** ledit procédé est utilisable dans la détection immunologique d'une pluralité d'entités et **en ce que** ledit procédé comprend l'utilisation d'une pluralité de paires liant-ligand auxiliaire différentes, le ligand auxiliaire de chacune des pluralités de paires liant-ligand auxiliaire étant fourni sur un matériau support, lesdits ligands auxiliaires n'étant pas des espèces primaires, ledit procédé étant tel qu'une interaction de liaison spécifique immunologique entre un ligand auxiliaire donné et un liant correspondant facilite l'association d'une espèce primaire avec un matériau support.

4. Procédé selon la revendication 3, **caractérisé en outre en ce que** ledit procédé comprend l'utilisation d'une pluralité de paires liant auxiliaire-ligand auxiliaire différentes, les ligands auxiliaires et les liants auxiliaires des paires n'étant pas des espèces primaires, et le ligand auxiliaire de chacune des pluralités de paires liant auxiliaire-ligand auxiliaire étant fourni sur un matériau support.

5. Capteur, **caractérisé en ce que** ledit capteur est utilisable dans la détection immunologique d'une pluralité d'entités et **en ce que** ledit capteur comprend un matériau support, étant entendu qu'est fournie sur le matériau support, lorsque le capteur est utilisé, une pluralité de ligands auxiliaires différents, lesdits ligands auxiliaires n'étant pas une espèce primaire, la disposition étant telle que, lorsque le capteur est utilisé, une interaction de liaison spécifique immunologique entre un ligand auxiliaire donné et un liant correspondant facilite l'association d'une espèce primaire avec le matériau support.

6. Procédé ou capteur selon l'une quelconque des revendications précédentes, **caractérisé en outre en ce qu'**un ligand auxiliaire est le 2,4-dinitrophénol, la fluorescéine, la digitoxine, la coumarine, le bleu de cibacron (marque déposée), l'hémiglutarate de 2-(4-aminophényl)-6-méthylbenzothiazole, l'acide camphorcarboxylique, le 4-aminobenzo-15-crown-5, la carboxyfluorescéine, l'acide 3-méthyl-1-adamantaneacétique, l'acide 2-phényl-4-quinoléinecarboxylique, la xanthine-9-carboxyamide-glycine-glycine, l'acide 4-hydroxy-7-trifluorométhyl-3-quinaldinecarboxylique, l'acide cis-bicyclo[3,3,0]octane-2-carboxylique, l'anhydride endo-bicyclo[2,2,2]oct-5-ène-2,3-dicarboxylique, le N-[4-(4-aminobenzyl)phényl]-5-norbornène-2,3-dicarboximide ou l'acide [IR-(2-endo, 3-exo)]-3-hydroxy-4,7,7-triméthylbicyclo[2,2,1]heptane-2-acétique.

7. Procédé ou capteur selon l'une quelconque des revendications 1 à 5, **caractérisé en outre en ce qu'**un ligand auxiliaire est fourni sous la forme d'un revêtement d'un polymère sur un matériau support.

8. Procédé ou capteur selon l'une quelconque des revendications 1 à 7, **caractérisé en outre en ce que** le liant est un liant auxiliaire et le liant auxiliaire est un anticorps anti-2,4-dinitrophénol, un anticorps antifluorescéine, un anticorps antidigitoxine, un anticorps anti-coumarine, un anticorps anti-bleu de cibacron, un anticorps anti-hémiglutarate de 2-(4-aminophényl)-6-méthylbenzothiazole, un anticorps anti-acide camphorcarboxylique, un anticorps anti-4-aminobenzo-15-crown-5, un anticorps anti-carboxyfluorescéine, un anticorps anti-acide 3-méthyl-1-adamantaneacétique, un anticorps anti-acide 2-phényl-4-quinoléinecarboxylique, un anticorps anti-xanthine-9-carboxyamide-glycine-glycine, un anticorps anti-acide 4-hydroxy-7-trifluorométhyl-3-quinaldinecarboxylique, un anticorps anti-acide cis-bicyclo[3,3,0]octane-2-carboxylique, un anticorps antianhydride endo-bicyclo[2,2,2]oct-5-ène-2,3-dicarboxylique, un anticorps anti-N-[4-(4-aminobenzyl)phényl]-5-norbornène-2,3-dicarboximide ou un anticorps anti-acide [IR-(2-endo, 3-exo)]-3-hydroxy-4,7,7-triméthylbicyclo[2,2,1]heptane-2-acétique.

9. Procédé ou capteur selon l'une quelconque des revendications 1 à 5, **caractérisé en outre en ce qu'**un liant est une espèce qui possède une partie susceptible de fournir une fonction auxiliaire.

10. Procédé selon la revendication 9, **caractérisé en outre en ce qu'**un liant est un anticorps bifonctionnel.

11. Procédé ou capteur selon l'une quelconque des revendications précédentes, **caractérisé en outre en ce que** différentes régions ou zones d'un matériau support sont disposées pour fournir des ligands auxiliaires différents, de sorte que des séparations spécifiques différentes peuvent être effectuées sur des régions ou zones différentes du matériau support.

12. Procédé ou capteur selon l'une quelconque des revendications précédentes, **caractérisé en outre en ce que** le matériau support lui-même ou une partie du matériau support lui-même fournit un ligand auxiliaire.

13. Procédé ou capteur selon l'une quelconque des revendications 1 à 11, **caractérisé en outre en ce qu'**un ligand auxiliaire est fixé à un matériau support.

14. Procédé ou capteur selon l'une quelconque des revendications 1 à 11 ou selon la revendication 13, **caractérisé en outre en ce qu'**un ligand auxiliaire est fixé directement à un matériau support.

15. Procédé ou capteur selon l'une quelconque des revendications 1 à 11 ou selon la revendication 13, **caractérisé en outre en ce qu'**un ligand auxiliaire est fixé indirectement à un matériau support.

16. Procédé ou capteur selon l'une quelconque des revendications 1 à 11, **caractérisé en outre en ce qu'**un oligomère d'un ligand auxiliaire ou un polymère d'un ligand auxiliaire est fixé directement ou indirectement à un matériau support.

17. Procédé ou capteur selon l'une quelconque des revendications précédentes, **caractérisé en outre en ce qu'**un matériau support est une paroi de récipient réactionnel, un polysaccharide insoluble, une microparticule, du polystyrène, du dextrane réticulé, une structure polymère insoluble, une surface de verre, une surface de silice dérivée, un polymère fixé à une surface, un matériau microparticulaire avec de l'oxyde ferreux piégé, du Nylon ou un polyamide.

18. Procédé selon l'une quelconque des revendications précédentes, **caractérisé en outre en ce que** ledit procédé comprend également l'étape consistant à fixer, directement ou indirectement, un ligand auxiliaire à un matériau support.

19. Procédé ou capteur selon l'une quelconque des revendications 1 à 16 ou selon la revendication 18, **caractérisé en outre en ce qu'**un matériau support se présente sous la forme d'un porteur qui peut être déplacé à partir d'un moyen d'application d'échantillon, en vue d'appliquer un échantillon sur le porteur, à un moyen de détection pour permettre ainsi la détection successive d'une pluralité d'entités différentes.

20. Procédé ou capteur selon l'une quelconque des revendications précédentes, **caractérisé en outre en ce qu'**un ligand auxiliaire ou un liant auxiliaire est associé à une espèce primaire et que l'espèce primaire est un anticorps primaire.

21. Procédé ou capteur selon l'une quelconque des revendications 1 à 19, **caractérisé en outre en ce qu'**un ligand auxiliaire ou un liant auxiliaire est associé à une espèce primaire et que l'espèce primaire est un ligand.

22. Détection immunologique ou dosage immunologique, **caractérisé en ce que** ledit procédé de détection immunologique comprend un procédé de séparation selon la revendication 1 ou la revendication 2.

23. Procédé selon l'une quelconque des revendication 3, 4 ou 6 à 19, **caractérisé en outre en ce que** ledit procédé comprend une détection immunologique ou un dosage immunologique comprenant un dosage immunologique hétérogène.

24. Procédé selon l'une quelconque des revendications 3, 4 ou 6 à 19, **caractérisé en outre en ce que** ledit procédé comprend une détection immunologique ou un dosage immunologique comprenant un dosage immunologique homogène.

25. Procédé ou capteur selon l'une quelconque des revendications précédentes, **caractérisé en outre en ce qu'**il comprend une espèce détectable.

26. Procédé ou capteur selon la revendication 25, **caractérisé en outre en ce qu'**une espèce détectable est une enzyme, une espèce capable de fournir un signal fluorescent, un composé chimioluminescent, un composé bioluminescent, un colorant ou un radioisotope.

27. Procédé ou capteur selon la revendication 25, **caractérisé en outre en ce qu'**une espèce détectable est une espèce de ligand, un polymère d'une espèce de ligand, une espèce de liant, ou un polymère d'une espèce de liant.

28. Procédé ou capteur selon la revendication 27, **caractérisé en outre en ce qu'**un espèce de ligand peut être détectée par une espèce de liant associée à un espèce de marqueur.

29. Procédé ou capteur selon la revendication 27, **caractérisé en outre en ce qu'**une espèce de liant peut être détectée par une espèce de ligand associée à une espèce de marqueur.

30. Procédé ou capteur selon la revendication 28 ou 29, **caractérisé en outre en ce que** l'espèce de marqueur est une enzyme.

31. Procédé ou capteur selon la revendication 28 ou la revendication 29, **caractérisé en outre en ce que** l'espèce de marqueur est une espèce capable de fournir un signal fluorescent, un composé chimioluminescent, un composé bioluminescent, un radioisotope ou un colorant.

32. Procédé ou capteur selon la revendication 28, **caractérisé en outre en ce que** l'espèce de ligand est une espèce antigénique.

33. Procédé ou capteur selon la revendication 28, **caractérisé en outre en ce que** l'espèce de ligand est une espèce de ligand non antigénique.

34. Procédé ou capteur selon la revendication 29, **caractérisé en outre en ce que** l'espèce de liant est un anticorps.

35. Procédé ou capteur selon la revendication 29, **caractérisé en outre en ce que** l'espèce de liant est une espèce de liant d'une espèce de ligand non antigénique.

36. Procédé ou capteur selon l'une quelconque des revendications précédentes, **caractérisé en outre en ce que** les entités à détecter sont elles-mêmes une espèce d'analyte.

37. Procédé ou capteur selon l'une quelconque des revendications 1 à 35, **caractérisé en outre en ce que** les entités à détecter portent ou comprennent une espèce d'analyte.

38. Procédé ou capteur selon la revendication 36, **caractérisé en outre en ce que** les entités à détecter sont des hormones stéroïdiennes, des hormones thyroïdiennes, des stéroïdes en extraits, des médicaments, des hormones polypeptidiques, des marqueurs tumoraux, des antigènes protéinés, des protéines du sang, des protéines marqueuses, des pesticides, des toxines, des micro-organismes, des anticorps dirigés contre des micro-organismes, ou des complexes de métaux.

39. Procédé ou capteur selon la revendication 37, **caractérisé en outre en ce que** l'espèce d'analyte est constituée par des ions métal.

40. Procédé selon l'une quelconque des revendications précédentes, **caractérisé en outre en ce qu'**un système de réactif reconstituable ou un matériau réactif reconstituable est utilisé.

41. Appareil **caractérisé en ce que** ledit appareil est utilisable dans la détection immunologique d'une pluralité d'entités et **en ce que** ledit appareil comprend un matériau support ou une pluralité de matériaux supports, étant entendu qu'est fournie sur le matériau support ou les matériaux supports, lorsque l'appareil est utilisé, une pluralité de ligands auxiliaires différents, lesdits ligands auxiliaires n'étant pas une espèce primaire, la disposition étant telle que, lorsque l'appareil est utilisé, une interaction de liaison spécifique immunologique entre un ligand auxiliaire donné et un liant correspondant facilite l'association d'une espèce primaire avec un matériau support.

42. Kit de dosage, **caractérisé en ce que** ledit kit de dosage est utilisé dans la détection immunologique d'une pluralité d'entités et **en ce que** ledit kit de dosage comprend un matériau support ou une pluralité de matériaux supports, étant entendu qu'est fournie sur le matériau support ou sur les matériaux supports, lorsque le kit de dosage est utilisé, une pluralité de ligands auxiliaires différents, lesdits ligands auxiliaires n'étant pas une espèce primaire, la disposition étant telle que, lorsque le kit de dosage est utilisé, une interaction de liaison spécifique immunologique entre un ligand auxiliaire donné et un liant correspondant facilite l'association d'une espèce primaire avec un matériau support.
